# EUROPEAN PATENT APPLICATION

(11) **EP 4 483 874 A1**
(43) Date of publication of application: **01.01.2025**
(21) Application number: 23756702.9
(22) Date of filing: 21.02.2023
(51) Int. Cl.: A61K 31/4035, A61P 25/28, A23L 33/10, A61K 45/06, A61P 31/04, A23K 20/195, A23K 20/132

(54) **NOVEL COMPOUND, AND COMPOSITION FOR PREVENTING, ALLEVIATING OR TREATING BACTERIAL INFLAMMATION AND INFLAMMASOME-MEDIATED DISEASES, COMPRISING SAME AS ACTIVE INGREDIENT**

(30) Priority: 21.02.2022 KR 20220022320; 27.10.2022 KR 20220140639; 04.11.2022 KR 20220146145
(71) Applicant: Quorum Bio Co., Ltd, Seoul 03080 (KR)
(72) Inventor: SIM, Jae Hyun, Seoul 07988 (KR); LI, Linzi, Seoul 08758 (KR); RYU, Eunju, Seoul 07703 (KR); KIM, Yunji, Seoul 01701 (KR); KIM, Hye Eun, Seoul 05033 (KR); YIM, Sangeun, Seoul 05649 (KR)
(74) Representative: Biggi, Cristina
(86) International application number: PCT/KR2023/002477
(87) International publication number: WO 2023/158290

(57) **Abstract**

The present application relates to: a compound of chemical formula 1, an isomer thereof or a pharmaceutically acceptable salt thereof; and use thereof for preventing, alleviating and/or treating inflammasome-mediated diseases and/or Gram-negative bacteria infections or diseases caused by Gram-negative bacteria, does not exhibit cell cytotoxicity, and thus is safe, and can effectively inhibit inflammasomes, thereby having the excellent effects of preventing, alleviating and/or treating these diseases.

## Description

### [TECHNICAL FIELD]

### CROSS-REFERENCE TO RELATED APPLICATION(S)

This application claims the benefit of Korean Patent Application No. 10-2022-0022320 filed on February 21, 2022, Korean Patent Application No. 10-2022-0140639 filed on October 27, 2022, Korean Patent Application No. 10-2022-0146145 filed on November 4, 2022 and Korean Patent Application No. 10-2023-0022804 filed on February 21, 2023 in the Korean Intellectual Property Office, the disclosures of which are incorporated herein by reference in their entirety.

### [BACKGROUND ART]

Gram-negative bacterium is known to cause periodontal disease, urinary tract infection, cystitis, pyelonephritis, abdominal infection, pneumonia, etc., and have a high virulence upon the human body and thus often lead to a secondary infection such as nosocomial infection and opportunistic infection. If bacteria infected in a local site are not controlled properly in an early stage, the virulence of bacteria increases, eventually circulating in the bloodstream and leading to a systemic inflammatory response (sepsis), which can lead to organ failure and shock death.

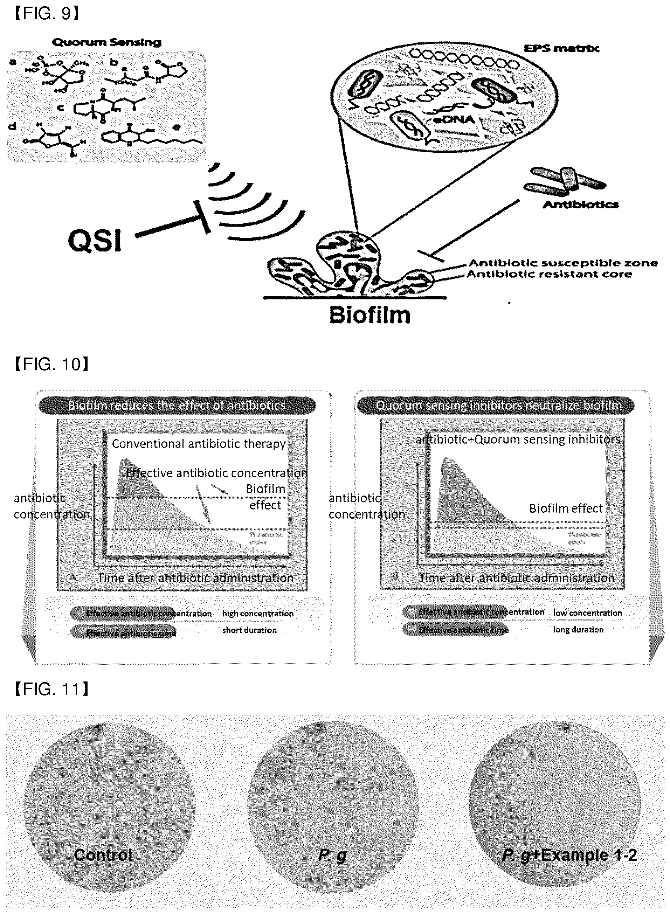

In the case of Gram-negative bacteria, they have an outer membrane that is not present in Gram-positive bacteria and thus are basically difficult for drugs to penetrate, and because of the bacterial membrane developed by quorum sensing in Gram-negative bacteria, high concentrations of antibiotics are required, and the effective period of the drug after administration also is short, which may lead to antibiotic overuse and resistance problems. However, by suppressing bacterial membrane formation of Gram-negative bacteria using a quorum sensing inhibitor and controlling the virulence that bacteria have, it is possible to fundamentally prevent the progression to related diseases (see FIGS. 9 and 10).

In particular, when a quorum sensing inhibitor is administered together with an antibiotic, the efficacy of the antibiotic is increased by offsetting the bacterial membrane effect, thereby lowering the effective concentration and extending the effective period, which may increase the antibiotic effect and minimize the induction of drug resistance.

There is a need to develop a novel therapeutic agent that targets Gram-negative bacterium (e.g., *Escherichia coli* (*E. coli*), *Pseudomonas aeruginosa* (*P. aeruginosa*)*, Acinetobacter baumannii (A. baumannii*), *Porphyromonas gingivalis* (*P. gingivalis*)*,* etc.), which causes various types of inflammation, such as sepsis which is a systemic inflammatory response syndrome, as well as periodontitis, osteomyelitis, urinary tract infections, cystitis, abdominal infections, or pneumonia,

Meanwhile, the inflammasome inflammatory complex may be activated due to causes such as Gram-negative bacterial infection to induce an immune response, and excessively activated NLRP3 inflammasome causes various inflammasome-mediated diseases such as neurodegenerative diseases, autoinflammatory diseases, and cancer. Therefore, there is a need to develop a therapeutic agent that can effectively treat diseases by inhibiting the NLRP3 inflammasome.

### [DETAILED DESCRIPTION OF THE INVENTION]

### [Technical Problem]

An embodiment of the present application provides a novel compound of the following Chemical Formula 1, an isomer thereof, and a pharmaceutically acceptable salt thereof:

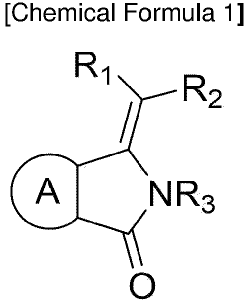

Another embodiment provides a composition (pharmaceutical composition, food composition, feed compositions, etc.) for preventing, alleviating and/or treating inflammasome-mediated diseases; and/or Gram-negative bacterial infections or diseases caused by Gram-negative bacteria, for protecting nerve cells and/or regenerating (or producing) nerve cells, comprising one or more kinds selected from the group consisting of the compound of Chemical Formula 1, an isomer thereof, and a pharmaceutically acceptable salt thereof as an active ingredient.

Another embodiment provides a use of one or more kinds selected from the group consisting of the compound of Chemical Formula 1, an isomer thereof, and a pharmaceutically acceptable salt thereof for preventing, alleviating and/or treating inflammasome-mediated diseases; and/or Gram-negative bacterial infections or diseases caused by Gram-negative bacteria, for protecting nerve cells, and/or regenerating (or producing) nerve cells.

Some embodiments provide a use of one or more kinds selected from the group consisting of the compound of Chemical Formula 1, an isomer thereof, and a pharmaceutically acceptable salt thereof for preparing a composition (pharmaceutical composition, food composition, feed compositions, etc.) for preventing, alleviating and/or treating inflammasome-mediated diseases; and/or Gram-negative bacterial infections or diseases caused by Gram-negative bacteria, for protecting nerve cells and/or regenerating (or producing) nerve cells.

Some embodiments provide a method for preventing, alleviating and/or treating inflammasome-mediated diseases; and/or Gram-negative bacterial infections or diseases caused by Gram-negative bacteria, a method for protecting nerve cells, and/or a method for regenerating or producing nerve cells, the method comprising administering an effective amount of one or more kinds selected from the group consisting of the compound of Chemical Formula 1, an isomer thereof, and a pharmaceutically acceptable salt thereof to a subject (individual) in need of prevention, amelioration and/or treatment of inflammasome-mediated diseases; and/or Gram-negative bacterial infections or diseases caused by Gram-negative bacteria, protection of nerve cells and/or regeneration or production of nerve cells.

Some embodiments provide an antibiotic comprising the compound of Chemical Formula 1. The antibiotic may exhibit antibiotic activity against Gram-negative bacterium.

The antibiotic provided herein (first antibiotic) is used in combination with another antibiotic (second antibiotic), and thereby has the advantages capable of exhibiting the effect of increasing antibiotic sensitivity of bacteria, increasing the bactericidal effect of the second antibiotic due to the bacteriostatic action of the first antibiotic itself, and exerting excellent antibiotic effects even by use of the second antibiotic at a low concentration. Consequently, it is possible to reduce side effects such as toxicity (e.g., nephrotoxicity, hepatotoxicity, etc.) caused by use of an antibiotic at a high concentration. In addition, it is possible to inhibit the appearance of antibioticresistant bacteria through the combined use of antibiotics with different mechanisms.

In this regard, one embodiment provides a combination antibiotic comprising:
compound; and
a second antibiotic

Some embodiments provide a feed additive comprising the antibiotic or the combination antibiotic as an active ingredient.

Some embodiments provide a disinfectant comprising the antibiotic or the combination antibiotic as an active ingredient.

Some embodiments provide a method for disinfection, comprising applying the antibiotic or the combination antibiotic to a subject in need of disinfection.

Some embodiments provide a cleaning agent comprising the antibiotic or the combination antibiotic as an active ingredient.

Some embodiments provide a cleaning method comprising applying the antibiotic or the combination antibiotic to a subject in need of cleaning.

### [Technical Solution]

An embodiment of the present application provides a compound of the following Chemical Formula 1, an isomer thereof, and a pharmaceutically acceptable salt thereof:

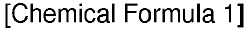

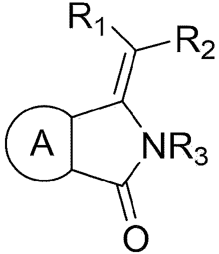

### Definition of Terms

The terms used herein are briefly explained below.

As used herein, the term "pharmaceutically acceptable salt" refers to a salt of a compound that does not cause significant irritation to an organism to which the compound is administered, and does not abrogate the biological activity and properties of the compound, and in the present invention, it collectively refers to any salt that equally retains the biological effectiveness and properties of the compound of Chemical Formula 1 and is preferred in terms of pharmaceutical, biological, or other properties. The pharmaceutically acceptable salt may include acid addition salts formed by acids capable of forming a non-toxic acid addition salt containing pharmaceutically acceptable anions, for example, inorganic acids such as hydrochloric acid, sulfuric acid, nitric acid, phosphoric acid, hydrobromic acid, hydroiodic acid, etc.; organic carbonic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc.; or sulfonic acids such as methanesulfonic acid, ethanesulfonic acid, benzenesulfonic acid, p-toluenesulfonic acid, etc. As a specific example thereof, an acid addition salt of the compound of one embodiment may be prepared by reacting free base forms of these compounds with a stoichiometric amount of the appropriate acid. At this time, the reaction may be performed in water, an organic solvent or a mixture thereof, and specifically, in a non-aqueous medium such as ether, ethyl acetate, ethanol, isopropanol, acetonitrile, etc. In addition, according to the form of the pharmaceutically acceptable salt, each form of the salts can be obtained by a typical reaction which is apparent to those skilled in the art. Further, the pharmaceutically acceptable salt may be alkaline metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline, triethylamine, etc.

As used herein, the term "isomer" means a compound or a salt thereof that has the same chemical formula or molecular formula, but is optically or geometrically different. The isomer, salt thereof, and mixture thereof (racemic mixture) are also included in the scope of the present invention.

As used herein, the term "aryl" refers to a carbocyclic group (e.g., phenyl) that has a covalent pi electron system and has at least one ring. The term includes a monocyclic or fused polycyclic (i.e., rings that share adjacent pairs of carbon atoms) group.

As used herein, the term "heteroaryl" refers to a heterocyclic aryl group that has a covalent pi electron system and has at least one ring, and represents, for example, furan, thiophene, pyrrole, imidazole, oxazole, isoxazole, oxadiazole, tetrazole, thiazole, imidazole, pyrazole, isothiazole, triazole, thiadiazole, pyridine, pyridazine, pyrimidine, pyrazine, triazine, etc., but are not limited thereto.

The aryl and/or the heteroaryl may be a substituted or unsubstituted C5-10, which may be an aryl and/or heteroaryl having: 5 to 10 carbon atoms (C5-C10), 5 to 9 carbon atoms (C5-C9), 5 to 8 carbon atoms (C5-C8), 5 to 7 carbon atoms (C5-C7), 5 to 6 carbon atoms (C5-C6), 6 to 10 carbon atoms (C6-C10), 6 to 9 carbon atoms (C6-C9), 6 to 8 carbon atoms (C6-C8), 6 to 7 carbon atoms (C6-C7), 5 carbon atoms, 6 carbon atoms, 7 carbon atoms, 8 carbon atoms, 9 carbon atoms, or 10 carbon atoms.

As used herein, the term "alkyl" refers to an aliphatic hydrocarbon group. An alkyl moiety may be "saturated alkyl" which does not include an alkenyl or alkynyl moiety, or "unsaturated alkyl" which includes at least one alkenyl or alkynyl moiety. The "alkene" moiety refers to a group containing at least one carbon-carbon double bond, and the "alkyne" moiety refers to a group containing at least one carbon-carbon triple bond. For example, the "alkyl" may be a saturated alky or an unsaturated alkyl having a linear, branched, or cyclic structure. Typical alkyl groups include, but are not limited to, methyl, ethyl, propyl, isopropyl, butyl, isobutyl, t-butyl, pentyl, hexyl, ethenyl, propenyl, butenyl, etc. For example, C1-C4-alkyl has 1 to 4 carbon atoms in the alkyl chain, and is selected from the group consisting of methyl, ethyl, propyl, iso-propyl, n-butyl, iso-butyl, sec-butyl and t-butyl.

The alkyl group used herein may be a C₁₋₁₀ linear or branched alkyl group, and the alkoxy group may be a C₁₋₁₀ linear or branched alkoxy group. The C₁₋₁₀ linear or branched alkyl group, or the C₁₋₁₀ linear or branched alkoxy group may be each independently a group having: 1 to 10 carbon atoms (C1-C10), 1 to 9 carbon atoms (C1-C9), 1 to 8 carbon atoms (C1-C8), 1 to 7 carbon atoms (C1-C7), 1 to 6 carbon atoms (C1-C6), 1 to 5 carbon atoms (C1-C5), 1 to 4 carbon atoms (C1-C4), 1 to 3 carbon atoms (C1-C3), 1 to 2 carbon atoms (C1-C2), 1 carbon number (C1), 2 to 10 carbon atoms (C2-C10), 2 to 9 carbon atoms (C2-C9), 2 to 8 carbon atoms (C2-C8), 2 to 7 carbon atoms (C2-C7), 2 to 6 carbon atoms (C2-C6), 2 to 5 carbon atoms (C2-C5), 2 to 4 carbon atoms (C2-C4), 2 to 3 carbon atoms (C2-C3), 2 carbon atoms (C2), 3 to 10 carbon atoms (C3-C10), 3 to 9 carbon atoms (C3-C9), 3 to 8 carbon atoms (C3-C8), 3 to 7 carbon atoms (C3-C7), 3 to 6 carbon atoms (C3-C6), 3 to 5 carbon atoms (C3-C5), 3 to 4 carbon atoms (C3-C4), 3 carbon atoms (C3), 4 to 10 carbon atoms (C4-C10), 4 to 9 carbon atoms (C4-C9), 4 to 8 carbon atoms (C4-C8), 4 to 7 carbon atoms (C4-C7), 4 to 6 carbon atoms (C4-C6), 4 to 5 carbon atoms (C4-C5), 4 carbon atoms (C4), 5 to 10 carbon atoms (C5-C10), 5 to 9 carbon atoms (C5-C9), 5 to 8 carbon atoms (C5-C8), 5 to 7 carbon atoms (C5-C7), 5 to 6 carbon atoms (C5-C6), 5 carbon atoms (C5), 6 to 10 carbon atoms (C6-C10), 6 to 9 carbon atoms (C6-C9), 6 to 8 carbon atoms (C6-C8), 6 to 7 carbon atoms (C6-C7), 6 carbon atoms (C6), 7 to 10 carbon atoms (C7-C10), 7 to 9 carbon atoms (C7-C9), 7 to 8 carbon atoms (C7-C8), 7 carbon atoms (C7), 8 to 10 carbon atoms (C8-10), 8 to 9 carbon atoms (C8-C9), 9 carbon atoms (C9), 9 to 10 carbon atoms (C9-C10), or 10 carbon atoms (C10).

As used herein, the term "halo" or "halogen" refers to fluoro(-F), chloro (-CI), bromo (-Br), or iodo(-I).

As used herein, the term 'heterocycle' refers to a group in which a cyclic carbon is replaced by oxygen, nitrogen, sulfur, etc., and the group may contain any double bond. The heterocycle may be exemplified by pyrroline, pyrrolidine, tetrahydrofuran, imidazoline, imidazolidin, pyrazoline, pyrazolidine, pyran, piperidine, piperazine, morpholine, thiomorpholine, etc., but is not limited thereto.

The term "each independently substituted" means that when the number of replaceable hydrogen atoms is two or more, each hydrogen can be replaced by the same or different substituent.

As used herein, the term "prevention" refers to any action that inhibits or delays the onset of a disease (disorder) by administration of the composition according to one embodiment, the term "treatment" refers to any action that ameliorates or beneficially change symptoms of suspected and onset individuals by administration of the composition according to one embodiment and the term "amelioration" refers to any action that reduces at least one parameter related to a condition to be treated, for example, the severity of a symptom. The disease may be an inflammasome-mediated disease.

As used herein, the term "pharmaceutically effective amount" means an amount of the active ingredient that is enough to obtain a desired pharmaceutical effect, and according to circumstances, it means a concentration or administration dose of the active ingredient in the pharmaceutical composition, which is enough for the desired pharmaceutical effect.

Terminology other than those described above may be construed as meaning commonly understood by those skilled in the art to which the present invention pertains.

### Compound of Chemical Formula 1

One embodiment of the present application provides a compound of the following Chemical Formula 1, an isomer thereof, and a pharmaceutically acceptable salt thereof: 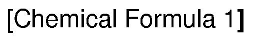 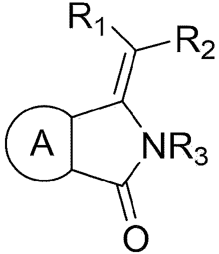 wherein in Chemical Formula 1,
the 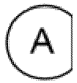 may be a substituted or unsubstituted C₃₋₇ cycloalkyl, a substituted or unsubstituted C₃₋₇ cycloalkenyl containing one or more double bonds, a substituted or unsubstituted C₃₋₇ heterocycloalkyl containing one or more heteroatoms selected from the group consisting of N, O, and S, a substituted or unsubstituted C₃₋₇ heterocycloalkenyl containing one or more double bonds and containing one or more heteroatoms selected from the group consisting of N, O, and S, a substituted or unsubstituted C₆₋₁₀ aryl, or a substituted or unsubstituted C₅₋₁₀ heteroaryl containing one or more heteroatoms selected from the group consisting of N, O, and S. In one specific embodiment, the 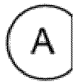 may be a substituted or unsubstituted C₆₋₁₀ aryl.

The substituted cycloalkyl, the substituted cycloalkenyl, the substituted heterocycloalkyl, the substituted heterocycloalkenyl, the substituted aryl, or the substituted heteroaryl may be substituted with one or more substituents selected from the group consisting of hydroxy(-OH), halogen(-F, -Br, -Cl, or -I), cyano(-CN), nitro(-NO₂), amino(-NH), a substituted or unsubstituted C₁₋₁₀ linear or branched alkyl, and a substituted or unsubstituted C₁₋₁₀ linear or branched alkoxy. (As used herein, "substituted with one or more" includes when substituted with two or more identical substituents: for example, when substituted with two OH, etc.).

The substituted alkyl or the substituted alkoxy may be substituted with one or more substituents selected from the group consisting of hydroxy(-OH), halogen, cyano(-CN), nitro(-NO₂), amino(-NH), a C₁₋₅ linear or branched alkyl, and a C₁₋₅ linear or branched alkoxy.

The R₃ may be a substituted or unsubstituted C₁₋₁₀ linear or branched alkyl, a substituted or unsubstituted C₁₋₁₀ linear or branched chain alkoxy, a substituted or unsubstituted C₃₋₇ cycloalkyl, a substituted or unsubstituted C₃₋₇ heterocycloalkyl containing one or more heteroatoms selected from the group consisting of N, O, and S, a substituted or unsubstituted C₆₋₁₀ aryl, or a substituted or unsubstituted C₅₋₁₀ heteroaryl containing one or more heteroatoms selected from the group consisting of N, O, and S. In one specific embodiment, the R₃ may be a substituted or unsubstituted C₆₋₁₀ aryl.

The substituted alkyl may be substituted with one or more substituents selected from the group consisting of hydroxy(-OH), halogen, cyano(-CN), nitro(-NO₂), amino(-NH), a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing one or more heteroatoms selected from the group consisting of N, O, and S.

The substituted alkoxy, the substituted cycloalkyl, the substituted heterocycloalkyl, the substituted aryl, or the substituted heteroaryl may be substituted with one or more substituents selected from the group consisting of hydroxy(-OH), halogen, cyano(-CN), nitro(-NO₂), amino(-NH), a C₁₋₅ linear or branched alkyl, and a C₁₋₅ linear or branched alkoxy.

The R₁ and R₂ may be each independently (or both) hydrogen (H), halogen (-F, -Br, -Cl, or -I), a substituted or unsubstituted C₁₋₁₀ linear or branched alkyl, a substituted or unsubstituted C₁₋₁₀ linear or branched alkoxy, a substituted or unsubstituted C₆₋₁₀ aryl, or a substituted or unsubstituted C₅₋₁₀ heteroaryl containing one or more heteroatoms selected from the group consisting of N, O, and S. In one specific embodiment, the R₁ and R₂ may be each independently a substituted or unsubstituted C₆₋₁₀ aryl, or a substituted or unsubstituted C₅₋₁₀ heteroaryl containing one or more heteroatoms selected from the group consisting of N, O, and S. In one specific embodiment, the substituted or unsubstituted C₆₋₁₀ aryl, or the substituted or unsubstituted C₅₋₁₀ heteroaryl may be substituted with one or more substituents selected from the group consisting of a methyl group, an ethyl group, a methoxy group, and an ethoxy group.

The substituted alkyl may be substituted with one or more substituents selected from the group consisting of hydroxy(-OH), halogen, cyano(-CN), nitro(-NO₂), amino(-NH), a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing one or more heteroatoms selected from the group consisting of N, O, and S.

The substituted alkoxy, the substituted aryl, or the substituted heteroaryl may be substituted with one or more substituents selected from the group consisting of hydroxy(-OH), halogen, cyano(-CN), nitro(-NO₂), amino(-NH), a C₁₋₅ linear or branched alkyl, and a C₁₋₅ linear or branched alkoxy.

In one embodiment, the compound of Chemical Formula 1 may be one or more kinds selected from the group consisting of the following compounds:
1) 3-(diphenylmethylene)-2-methylisoindolin-1 -one,
2) 3-(di-p-tolylmethylene)-2-methylisoindolin-1-one, and
3) 3-(bis(4-methoxyphenyl)methylene)-2-methylisoindolin-1-one.

Some embodiments of the present application provide a compound of the following Chemical Formula 1A, an isomer thereof, or a pharmaceutically acceptable salt thereof: 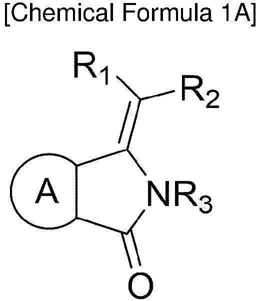 wherein in Chemical Formula 1A,
the 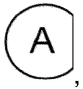 R₁, R₂ and R₃ are as defined above. However, the compound of Chemical formula 1A may not include the following compound:
3-(diphenylmethylene)-2-methylisoindolin-1-one (CAS No. 92172-54-8).

In one embodiment, the compound of Chemical Formula 1A may be one or more kinds selected from the group consisting of the following compounds:
1) 3-(di-p-tolylmethylene)-2-methylisoindolin-1-one, and
2) 3-(bis(4-methoxyphenyl)methylene)-2-methylisoindolin-1-one.

The isomer of the compound of Chemical Formula 1 or Chemical Formula 1A (hereinafter, the compound of Chemical Formula 1 or Chemical Formula 1A are uniformly described as a compound of Chemical Formula 1, unless otherwise specified) may be an optical isomer, a stereoisomer, or a mixture of isomers (racemic mixture) of the compound of Formula 1. In the compound of Chemical Formula 1 according to one embodiment, it is understood that each substituent may be attached to a chiral center of a carbon atom. Further, any asymmetric carbon atom on the compound of the one embodiment may exist in any form of (R)-, (S)- or (R, S)- configuration, and suitably in the (R)- or (S)-configuration, each of which is an isolated form. Further, the compound of one embodiment may exist in any of the possible isomeric forms or mixtures thereof, and for example, it may exist in any form as a pure geometric isomer, diastereomer, optical isomer, racemate, or mixtures thereof. In addition, when the compound of one embodiment has a double bond, each substituent attached to the double bond may be in the E or Z configuration.

### Inflammasome-mediated disease

As used herein, the term "inflammasome" refers to a caspase-1-activating multiprotein complex, and may include 1) NLR (nucleotide-binding oligomerization domain and leucine-rich repeat-containing receptor) protein, which is a sensor protein, 2) ASC (adaptor protein apoptosis-associated spec-like protein containing a caspase-recruitment domain), which is an adapter protein, and 3) pro-caspase-1, which is an effector protein. IL-1β, which is one of the inflammatory cytokines, is formed by a multiprotein polymer called inflammasome, and can mediate pyroptosis, which is a type of inflammatory apoptosis. In regard to inflammasome, when toll-like receptors(TLRs) are activated by pathogen-associated molecular pattern molecules(PAMP), which are substances possessed by pathogens, or damage-associated molecular pattern molecules(DAMP), which are produced by dead cells, the expression of IL-1β precursor (pro-IL-1β) increases, and subsequently, caspase-1 is activated by a protein complex called inflammasome to decompose the IL-1β precursor, thereby capable of producing activated IL-1β.

The inflammasome may be selected from the group consisting of NLRP3 (NOD-like receptor family, pyrin domain-containing 3) inflammasome, AIM2 (Absent in melanoma 2) inflammasome and NLRC4 (NLR family CARD domain-containing protein 4) inflammasome, but is not limited thereto. The NLRP3, AIM2, or NLRC4 inflammasome is meant to include nucleic acids, polynucleotides, oligonucleotides, sense and anti-sense polynucleotide strands, complementary sequences, peptides, polypeptides, proteins, homologous and/or orthologous molecules, isoforms, precursor, mutants, variants, derivative, splice variants, alleles, different species, and active fragments thereof, but is not limited thereto.

If the inflammasome is excessively activated, inflammasome-mediated disease may occur. Activation of the inflammasome may be caused by Gram-negative bacterial infections or Gram-negative bacteria, but is not limited thereto. In one embodiment, the compound of the present application can inhibit the activation of inflammasome (e.g., inhibiting the production of inflammatory cytokine IL-1β, etc.) to effectively regulate the secretion amount of the inflammatory cytokine IL-1β, thereby exhibiting excellent effects in preventing, alleviating, or treating inflammasome-mediated diseases.

The inflammasome-mediated disease may refer to any type of diseases that occurs when inflammasome is abnormally and excessively activated.

In one embodiment, the inflammasome-mediated disease may be a periodontal disease.

In one embodiment, the inflammasome-mediated disease may be a neurodegenerative disease.

In one embodiment, the inflammasome-mediated disease may be an inflammatory disease. The inflammatory disease may be an inflammasome-mediated inflammatory disease.

In one embodiment, the inflammasome-mediated disease may be cancer.

In one embodiment, the inflammasome-mediated disease may be a Gram-negative bacterial infection or a disease caused by Gram-negative bacteria.

In one embodiment, the inflammasome-mediated disease may be one or more kinds selected from the group consisting of periodontal disease, neurodegenerative disease, inflammatory diseases (e.g., inflammasome-mediated inflammatory disease), cancer, Gram-negative bacterial infection or disease caused by Gram-negative bacteria, metabolic disease, sepsis, septic shock, inflammatory bowel diseases, osteoarthritis, hyperimmunoglobulin D syndrome, and cryopyrin-associated periodic syndromes, but is not limited thereto.

The compound of the present application exhibits antibiotic effects against Gram-negative bacteria, specifically, inhibitory activity against virulence in Porphyromonas sp. bacterium, as described below, and may have prophylactic, ameliorative, and/or therapeutic activity against periodontal disease.

The inhibitory activity against virulence in Gram-negative bacteria may be achieved through quorum sensing inhibition by Gram-negative bacteria, and/or expression inhibition of virulence factors (e.g., biofilm of Gram-negative bacteria, or gingipain, etc. in the case of Porphyromonas sp. bacterium) in Gram-negative bacteria (e.g., suppression of the mRNA transcription process, or suppression of the protein translation process, etc.), but is not limited thereto. The gingipain may be one or more kinds selected from the group consisting of Lysine-gingipain (Kgp), Arginine-gingipain A (RgpA), Arginine-gingipain B (RgpB), etc., but is not limited thereto.

The prophylactic, ameliorative, and/or therapeutic effects of periodontal disease can be achieved through the inhibition of Gram-negative bacterial virulence. That is, the prevention, amelioration and/or treatment of periodontal disease includes inhibition of Gram-negative bacterial virulence, for example, quorum sensing inhibition by Porphyromonas sp. bacterium, and/or inhibition of virulence factors (e.g., gingipain, biofilm, etc.) in Porphyromonas sp. bacterium, etc. (e.g., suppression of the mRNA transcription process or suppression of the protein translation process, etc.), but is not limited thereto.

The periodontal disease may be one or more diseases selected from periodontitis, gingivitis, etc.

The compound of the present application may be characterized by having the expression inhibition of inflammatory proteins (e.g., inflammatory cytokines, etc.) in patients with neurodegenerative diseases, and inhibitory activity against the inflammation and death of nerve cells (neuroinflammation, etc.) and/or the death and/or inflammation of neuronal cells caused by *P. gingivalis.*

As used herein, the neurodegenerative disease may be selected from among all diseases that show abnormalities in motor control ability, cognitive function, perceptual function, sensory function, and autonomic nerve function due to death, reduction, functional decline, or loss of function of nerve cells. For example, the neurodegenerative disease may be one or more types selected from the group consisting of dementia (e.g., Alzheimer's disease(AD), vascular dementia, mixed dementia, Lewy body dementia, frontotemporal dementia, etc.), Parkinson's disease(PD), Huntington's disease, amyotrophic lateral sclerosis (Lou Gehrig's disease), hand tremor, chorea, multiple sclerosis, motor neuron disease, spinal muscular atrophy, Creutzfeldt-Jakob disease, Pick's disease, Prion disease, spinocerebellar ataxia, etc., but is not limited thereto.

The neurodegenerative disease may be induced by infection with Gram-negative bacteria, such as Porphyromonas sp. bacterium, or may be induced by Porphyromonas sp. bacterium, but is not limited thereto. The Porphyromonas sp. bacterium may be one or more kinds selected from the group consisting of *Porphyromonas gingivalis, Porphyromonas endodontalis,* etc.

The nerve cells may be central nerve cells, e.g., brain (nerve) cells.

The inflammatory protein (e.g., inflammatory cytokine) may be IL-6, IL-8, IL-18, TNF-α, IL-1, etc., but is not limited thereto.

The inflammatory disease may refer to an abnormal inflammatory disease that occurs when inflammasome is abnormally and excessively activated, and may include a neuroinflammatory disease, an autoinflammatory disease, an autoimmune disease, etc.

The neuroinflammatory disease may refer to a disease caused by damage to nerve tissue due to an inflammatory response, and may be one or more kinds selected from the group consisting of Alzheimer's disease, Parkinson's disease, Huntington's disease, Lou Gehrig's disease, Creutzfeldt-Jakob disease, multiple sclerosis, amyotrophic lateral sclerosis, inflammatory spinal injury, diffuse Lewy body disease, leukoencephalitis, temporal lobe epilepsy, and inflammatory spinal cord injury, but is not limited thereto.

The autoinflammatory disease may be one or more kinds selected from the group consisting of Muckle-Wells syndrome(MWS), latent autoimmune diabetes in adults (LADA), familial cold autoinflammatory syndrome(FCAS), cryopyrin-associated periodic syndrome(CAPS), neonatal-onset multisystem inflammatory syndrome(NOMID), chronic infantile neurocutaneous articular(CINCA) syndrome, Familial Mediterranean fever(FMF), juvenile arthritis (e.g., systemic juvenile idiopathic arthritis(SJIA)), juvenile rheumatoid arthritis (e.g., systemic juvenile idiopathic rheumatoid arthritis), and gout, but is not limited thereto.

The autoimmune disease may be one or more kinds selected from the group consisting of rheumatoid arthritis(RA), systemic lupus erythematosus(SLE), atopic dermatitis(AD) and psoriasis, but is not limited thereto.

In addition, activation of the inflammasome is known to be involved in the pathogenesis of many cancers, wherein the cancer may be one or more kinds selected from the group consisting of large intestine cancer, acute myeloid leukemia, adrenal cortical carcinoma, Kaposi's sarcoma, lymphoma, anal cancer, appendix cancer, teratoid/ rhabdomyosarcoma, basal cell carcinoma, bile duct cancer, bladder cancer, bone cancer, brain cancer, breast cancer, bronchial tumor, carcinoid tumor, heart tumor, cervical cancer, chordoma, chronic lymphocytic leukemia, chronic myeloproliferative neoplasm, colon cancer, colorectal cancer, craniopharyngioma, endometrial cancer, ependymoma, esophageal cancer, sensory neuroblastoma, Ewing's sarcoma, eye cancer, fallopian tube cancer, gallbladder cancer, gastrointestinal carcinoid tumor, gastrointestinal stromal tumor, germ cell tumor, hairy cell leukemia, head and neck cancer, heart cancer, liver cancer, hypopharyngeal cancer, pancreatic cancer, kidney cancer, laryngeal cancer, chronic myeloid leukemia, lip cancer, oral cancer, lung cancer, melanoma, Merkel cell carcinoma, mesothelioma, intraoral cancer, oral cancer, osteosarcoma, ovarian cancer, penile cancer, pharyngeal cancer, prostate cancer, rectal cancer, salivary gland cancer, skin cancer, small intestine cancer, soft tissue sarcoma, testicular cancer, throat cancer, thyroid cancer, urethral cancer, uterine cancer, vaginal cancer, and vulvar cancer, but is not limited thereto.

The compound of the present application can inhibit inflammasome to effectively prevent, ameliorate, or treat the cancer.

The metabolic disease is collectively referred to as diseases caused by metabolic disorders in the living body, and may be one or more kinds selected from the group consisting of obesity, hyperlipidemia, hypercholesterolemia, arteriosclerosis, type 2 diabetes, non-alcoholic fatty liver disease(NAFLD), and non-alcoholic steatohepatitis(NASH), but is not limited thereto.

### Antibiotics against Gram-negative bacteria and Gram-negative bacterial infections or diseases caused by Gram-negative bacteria

Yet another embodiment provides an antibiotic comprising the above compound.

Some embodiments provide a use of the compound as an antibiotic against Gram-negative bacteria (e.g., inhibition (or suppression) of the growth of Gram-negative bacteria, bacteriostasis of Gram-negative bacteria, sterilization and pest control of Gram-negative bacteria, etc.).

Some embodiments provide a sterilization method (or bacteriostatic method, pest control method, or growth inhibition method) for gam-negative bacteria comprising a step of applying (or administering) an effective amount of the compound to a subject in need of antibiosis against Gram-negative bacteria (or bacteriostasis, sterilization, pest control, etc. of Gram-negative bacteria).

Some embodiments provide a use of the compound for the preparation of antibiotics.

The bacteriostatic effect may mean that an antibiotic irreversibly binds to bacteria and does not sterilize the bacteria, but inhibits their growth. The bactericidal effect may mean that an antibiotic irreversibly binds to bacteria and sterilizes the bacteria beyond inhibiting their growth. The distinction between bacteriostatic and bactericidal effects is not absolute, and they can use together and function as bacteriostatic or bactericidal agents depending on the concentration of the administered antibiotic.

The compound of Chemical Formula 1 may have (i) inhibitory activity against virulence in Gram-negative bacteria, (ii) antibiotic effect against Gram-negative bacterium (e.g., bacteriostatic effect and/or bactericidal effect), and/or (iii) prophylactic, ameliorative, and/or therapeutic activity against Gram-negative bacterial infections or diseases caused by Gram-negative bacteria.

The inhibitory activity against virulence in Gram-negative bacteria, or antibiotic effect against Gram-negative bacterium may be achieved through quorum sensing inhibition by Gram-negative bacteria, and/or expression inhibition of virulence factors (e.g., biofilm, extracellular polysaccharides (EPSs), toxins, etc.) in Gram-negative bacteria (e.g., suppression of the mRNA transcription process, or suppression of the protein translation process, etc.), but is not limited thereto.

The prophylactic, ameliorative, and/or therapeutic effects of Gram-negative bacterial infections or diseases caused by Gram-negative bacteria can be achieved through the inhibition of Gram-negative bacterial virulence or the antibiotic effect against Gram-negative bacterium. That is, the prevention, amelioration and/or treatment of Gram-negative bacterial infections or diseases caused by Gram-negative bacteria can be achieved through the inhibition of Gram-negative bacterial virulence, or the antibiotic effect against Gram-negative bacterium, for example, quorum sensing inhibition by Gram-negative bacteria, and/or inhibition of virulence factors (e.g., biofilm, extracellular polysaccharides (EPSs), toxins, etc.) in Gram-negative bacteria, etc. (e.g., suppression of the mRNA transcription process or suppression of the protein translation process, etc.), but is not limited thereto.

The antibiotic may have an antibiotic effect against Gram-negative bacterium. The Gram-negative bacterium may be one or more kinds (e.g., one, two, three, four, or five kinds) selected from the group consisting of Escherichia sp. bacterium, Pseudomonas sp. bacterium, Acinetobacter sp. bacterium, Enterobacter sp. bacterium, Klebsiella sp. bacterium, Porphyromonas sp. bacterium, Fusobacteria sp. bacterium, Tannerella sp. bacterium, and the like. For example. the Escherichia sp. bacterium may be *Escherichia coli*, the Pseudomonas sp. bacterium may be *Pseudomonas aeruginosa,* the Acinetobacter sp. bacterium may be *Acinetobacter baumannii,* the Enterobacter sp. bacterium may be *Enterobacter aerogenes* (also known as *Klebsiella aerogenes*) or (*Enterobacter cloacae*), the Klebsiella sp. bacterium may be *Klebsiella pneumoniae,* the Porphyromonas sp. bacterium may be *Porphyromonas gingivalis* or *Porphyromonas endodontalis,* the Fusobacteria sp. bacterium may be *Fusobacterium nucleatum,* and the Tannerella sp. bacterium may be *Tannerella forsythia,* but is not limited thereto.

The Gram-negative bacterial infections or the diseases caused by Gram-negative bacteria may be selected from all diseases caused by Gram-negative bacterial infections, and for example, they may be one or more kinds selected from the group consisting of enteritis, Crohn's disease, ulcerative colitis, bacillary dysentery, urinary tract infection, skin infection, bacteremia, sepsis, skin infections, bedsores, pneumonia, endocarditis, meningitis, otitis externa, otitis media, keratitis, osteomyelitis, peritonitis, periodontitis, cystic fibrosis, and periodontal disease (e.g., gingivitis, peritonitis, etc.), but is not limited thereto.

The Gram-negative bacterial infections or the diseases caused by Gram-negative bacteria may be selected from the group consisting of, for example, diseases caused by Escherichia sp. bacterium, such as enteritis, Crohn's disease, ulcerative colitis, bacillary dysentery, urinary tract infection, skin infection, and bacteremia, sepsis; diseases caused by Pseudomonas sp. bacterium, such as skin infections, bedsores, pneumonia, bacteremia, sepsis, endocarditis, meningitis, otitis externa, otitis media, keratitis, osteomyelitis, enteritis, periodontitis, and cystic fibrosis; diseases caused by Acinetobacter sp. bacterium such as skin infections, pneumonia, bacteremia, and sepsis; diseases caused by Porphyromonas sp. bacterium, Fusobacteria sp. bacterium, or Tannerella sp. bacterium such as gingivitis, periodontitis, etc., but is not limited thereto.

The antibiotics provided herein can be used in combination with other antibiotics (second antibiotics) in addition to the compounds described above.

Thus, a further aspect provides a combination antibiotic comprising the antibiotic and a second antibiotic.

The combination antibiotic may have an antibiotic effect against Gram-negative bacterium. The Gram-negative bacterium is the same as described above.

The second antibiotic may be one or more kinds selected from commonly used antibiotics, for example, antibiotics having antibiotic activity against Gram-negative bacterium. The second antibiotic may be one or more kinds selected from the group consisting of beta-lactam antibiotics, bacterial cell membrane permeability inhibitors, bacterial ribosome inhibitors, bacterial nucleic acid synthesis inhibitors, and bacterial folate synthesis inhibitors.

In one specific embodiment, the second antibiotic may be one or more kinds selected from the group consisting of:
beta-lactam antibiotics;
one or more bacterial cell membrane permeability inhibitors selected from the group consisting of polymicin, amphotericin B, ketoconazole, fluconazole, itraconazole, etc.;
one or more bacterial ribosome inhibitors selected from the group consisting of aminoglycoside, tetracycline, macrolide, lincosamide, chloramphenicol, etc.;
one or more bacterial nucleic acid synthesis inhibitors selected from the group consisting of quinolone, fluoroquinolone, rifampicin, etc.; and
one or more bacterial folate synthesis inhibitors selected from the group consisting of sulfonamide, trimethoprim, etc.

The beta-lactam antibiotics may include:
one or more penicillin antibiotics selected from the group consisting of penicilln G, meticillin, oxacillin, flucloxacillin, amoxicillin, ampicillin, ampicillin/sulbactam, piperacillin, piperacillin/tazobactam, ticarcillin, Azlocillin, Carbenicillin and amoxicillin/clavulanic acid, etc.;
one or more cephalosporin antibiotics selected from the group consisting of cefazolin, cephalexin, cephalothin, ceftezol, cefazedone, cefaclor, cefamandole, cefmetazole, cefotiam, cefuroxime, cefotaxime, ceftriaxone, ceftazidime and cefepime, etc. ;
monobactam antibiotics such as aztreonam; and
one or more carbapenem antibiotics selected from the group consisting of imipenem, imipenem/cilastatin, faropenem, meropenem, doripenem, and artapenem, but not limited thereto.

The second antibiotic may have improved antibiotic activity when used in combination with a beta-lactamase inhibitor such as clavulanic acid, sulbactam or tazobactam, or cilastatin.

In one embodiment, the second antibiotic may be used in combination with a substance such as clavulanic acid, sulbactam, tazobactam, and cilastatin, and for example, it can be used in combination with amoxicillin/clavulanic acid, ampicillin/sulbactam, piperacillin/tazobactam, imipenem/cilastatin, etc. At this time, the second antibiotic and clavulanic acid, sulbactam, tazobactam, cilastatin, etc. may be used in combination at various ratios, such as 1: 10 to 0.1, 1: 9 to 0.1, 1: 8 to 0.1, 1: 7 to 0.1, 1: 6 to 0.1, 1: 5 to 0.1, 1: 4 to 0.1, 1: 3 to 0.1, 1: 2 to 0.1, 1: 10 to 0.2, 1: 9 to 0.2, 1: 8 to 0.2, 1: 7 to 0.2, 1: 6 to 0.2, 1: 5 to 0.2, 1: 4 to 0.2, 1: 3 to 0.2, 1: 2 to 0.2, 1: 10 to 0.5, 1: 9 to 0.5, 1: 8 to 0.5, 1: 7 to 0.5, 1: 6 to 0.5, 1: 5 to 0.5, 1: 4 to 0.5, 1: 3 to 0.5, 1: 2 to 0.5, 1: 10 to 0.75, 1: 9 to 0.75, 1: 8 to 0.75, 1: 7 to 0.75, 1: 6 to 0.75, 1: 5 to 0.75, 1: 4 to 0.75, 1: 3 to 0.75, 1: 2 to 0.75, 1: 2, 1: 0.5 on a weight basis.

Further, the second antibiotic may be used in combination with two or more types of second antibiotics, and when used in combination with two or more types of the second antibiotic, the respective second antibiotics may be used in combination at various ratios, such as 1: 10 to 0.1, 1: 9 to 0.1, 1: 8 to 0.1, 1: 7 to 0.1, 1: 6 to 0.1, 1: 5 to 0.1, 1: 4 to 0.1, 1: 3 to 0.1, 1: 2 to 0.1, 1: 10 to 0.2, 1: 9 to 0.2, 1: 8 to 0.2, 1: 7 to 0.2, 1: 6 to 0.2, 1: 5 to 0.2, 1: 4 to 0.2, 1: 3 to 0.2, 1: 2 to 0.2, 1: 10 to 0.5, 1: 9 to 0.5, 1: 8 to 0.5, 1: 7 to 0.5, 1: 6 to 0.5, 1: 5 to 0.5, 1: 4 to 0.5, 1: 3 to 0.5, 1: 2 to 0.5, 1: 10 to 0.75, 1: 9 to 0.75, 1: 8 to 0.75, 1: 7 to 0.75, 1: 6 to 0.75, 1: 5 to 0.75, 1: 4 to 0.75, 1: 3 to 0.75, 1: 2 to 0.75, 1: 2, 1: 0.5 on a weight basis.

An antibiotic (first antibiotic) is used in combination with another antibiotic (second antibiotic) in this way, and thereby has the advantages capable of exhibiting the effect of increasing antibiotic sensitivity of bacteria, increasing the bactericidal effect of the second antibiotic due to the bacteriostatic action of the first antibiotic itself, and exerting excellent antibiotic effects even by use of the second antibiotic at a low concentration. Alternatively, due to the quorum sensing inhibition and/or biofilm formation inhibition and/or Gram-negative bacterial virulence attenuation effect that the first antibiotic has, when the first antibiotic and the second antibiotic are used in combination, there is an advantage that the second antibiotic, which has an antibiotic effect against Gram-negative bacterium, can exert an excellent antibiotic effect even at a low concentration. Consequently, it is possible to reduce side effects such as toxicity (e.g., nephrotoxicity, hepatotoxicity, etc.) caused by use of an antibiotic at a high concentration. In addition, it is possible to inhibit the emergence of antibioticresistant bacteria through the concomitant use of antibiotics with different mechanisms.

As used herein, the term "antibiotic" encompasses all types of agents that have growth inhibition ability (e.g., quorum sensing inhibition ability, biofilm formation inhibition ability, and/or virulence attenuation ability) and/or killing ability for Gram-negative bacteria, and it may be used interchangeably with antibacterial agents, preservatives, disinfectants, etc., unless otherwise specified.

### Pharmaceutical composition

A further aspect provides a pharmaceutical composition for preventing or treating inflammasome-mediated diseases, and/or Gram-negative bacteria infections or diseases caused by Gram-negative bacteria, comprising the compound, an isomer thereof, and a pharmaceutically acceptable salt thereof as an active ingredient.

Some aspects provide a use of one or more kinds selected from the group consisting of the compound of Chemical Formula 1, an isomer thereof, and a pharmaceutically acceptable salt thereof for preventing, alleviating and/or treating inflammasome-mediated diseases; and/or Gram-negative bacterial infections or diseases caused by Gram-negative bacteria.

Some aspects provide a pharmaceutical composition for preventing, alleviating and/or treating inflammasome-mediated diseases; and/or Gram-negative bacterial infections or diseases caused by Gram-negative bacteria, comprising one or more kinds selected from the group consisting of the compound of Chemical Formula 1, an isomer thereof, and a pharmaceutically acceptable salt thereof.

Some aspects provides a method for preventing, alleviating and/or treating inflammasome-mediated diseases; and/or Gram-negative bacterial infections or diseases caused by Gram-negative bacteria, the method comprising a step of administering an effective amount of one or more kinds selected from the group consisting of the compound of Chemical Formula 1, an isomer thereof, and a pharmaceutically acceptable salt thereof to a subject in need of prevention, amelioration and/or treatment of inflammasome-mediated diseases; and/or Gram-negative bacterial infections or diseases caused by Gram-negative bacteria. The method may, prior to the administering step, further comprise a step of identifying a subject in need of prevention, amelioration, and/or treatment of inflammasome-mediated diseases; and/or Gram-negative bacterial infections or diseases caused by Gram-negative bacteria.

Some aspects provide a use of one or more kinds selected from the group consisting of the compound of Chemical Formula 1, an isomer thereof, and a pharmaceutically acceptable salt thereof for the preparation of a composition for preventing, alleviating and/or treating inflammasome-mediated diseases; and/or Gram-negative bacterial infections or diseases caused by Gram-negative bacteria.

Some embodiments provide a pharmaceutical composition for protecting nerve cells, and/or a pharmaceutical composition regenerating or producing nerve cells, comprising the above-mentioned compound.

Some embodiments provide a method protecting nerve cells, and/or a method regenerating or producing nerve cells, comprising a step of administrating the above-mentioned compound to a subject in need of protecting nerve cells, and/or regenerating or producing nerve cells.

Some aspects provide a use of the above-mentioned compound for the preparation of a pharmaceutical composition for protecting nerve cells, and/or a pharmaceutical composition regenerating (or producing) nerve cells.

Hereinafter, unless otherwise specified, the compound of Chemical Formula 1 as an active ingredient of the pharmaceutical composition includes all isomers of the compound, and pharmaceutically acceptable salts of the compound or isomer, and all of these compounds, isomers, and salts should be construed as being included within the scope of the present application. However, for convenience of explanation, unless otherwise specified herein, one or more kinds selected from the group consisting of the compound of Chemical Formula 1, the isomer thereof, and the pharmaceutically acceptable salt thereof as the active ingredient of the pharmaceutical composition will be simply referred to 'compound of Chemical Formula 1'.

The inflammasome-mediated disease is the same as previously described.

The administration subject of the pharmaceutical composition may be one or more kinds selected from a mammal including primates such as human and monkeys, rodents such as mice and rats, livestock such as dogs, cats, pigs, cattle, horses, sheep and goats, poultry such as chickens, ducks, geese, pheasants, quails and turkeys, and the like, or a cell, tissue or culture thereof derived therefrom. For example, the administration subject may be a subject in need of prevention, amelioration and/or treatment of inflammasome-mediated diseases; and/or Gram-negative bacterial infections or diseases caused by Gram-negative bacteria.

In one embodiment, the administration subject (patient) may be selected from mammals including primates such as humans, apes, and the like and rodents such as rats, mice, and the like, which suffer from a neurodegenerative disease, cells (brain cells) or tissues (brain tissues) isolated from the mammals, or cultures thereof. By way of example, the subject may be selected from a human suffering from a neurodegenerative disease, brain cells or tissues isolated therefrom, or a culture of the cells or tissues.

A pharmaceutical composition comprising one or more kinds selected from the group consisting of the compound of Chemical Formula 1, the isomer thereof, and the pharmaceutically acceptable salt thereof as an active ingredient may be formulated and used in the form of a typical drug preparation. For example, the drug formulation may be prepared in a variety of formulations for oral or parenteral administration, and the type of the formulation may differ, depending on the use, administration method, administration purpose, etc. When prepared in a variety of formulations for oral or parenteral administration, it may be formulated by using one or more kinds selected from the group consisting of a diluent or an excipient, such as a filler, an extender, a binder, a wetting agent, a disintegrating agent, and a surfactant which are generally used. When preparing various preparations for oral or parenteral administration, the composition can be formulated by mixing one or more kinds selected from the group consisting of commonly used fillers, extenders, binders, wetting agents, disintegrating agents, diluents such as surfactant, or excipients.

When the pharmaceutical composition is administered orally, the composition should be coated or formulated for an active drug to be protected from degradation in stomach because protein or peptide is digested. Solid formulations for oral administration may include tablets, pills, powders, granules, capsules, and the like. Such solid formulations may be prepared by mixing the active ingredient with one or more excipients, e.g., one or more selected from the group consisting of starch, calcium carbonate, sucrose, lactose, gelatin, and the like. In addition to simple excipients, lubricants such as magnesium stearate and talc may also be used. Further, liquid formulations for oral administration may include suspensions, liquids for internal use, emulsions, syrups, and the like. When formulating into the liquid formulation, commonly used simple diluents such as water and/or liquid paraffin may be used, and optionally, one or more selected from the group consisting of various excipients, for example, wetting agents, sweeteners, fragrances, preservatives, and the like may be further included. Further, the composition can be administered by any device capable of transporting the active agent to target cells.

When the pharmaceutical composition is administered parenterally, the parenteral administration may be performed by any convenient route, such as vascular administration (e.g., arterial administration, intravenous administration, etc.), injection or insertion into the patient's lesion site (e.g., gums, etc.), intramuscular administration, subcutaneous administration, intraperitoneal administration, intranasal administration, transdermal administration, intradermal administration, topical administration, intrapulmonary administration, intrarectal administration, intravenous injection, subcutaneous injection, intramuscular injection, intraperitoneal injection, but is not limited thereto. Formulations for parenteral administration include sterilized aqueous solutions, non-aqueous solutions, suspensions, emulsions, lyophilized agents, suppositories, and the like. As the non-aqueous solvent for producing a non-aqueous solution or the suspension solvent for producing a suspension, propylene glycol, polyethylene glycol, vegetable oil such as olive oil, injectable ester such as ethyl oleate, and the like may be used. As a base for the suppository, witepsol, macrogol, tween 61, cacao butter, laurinum, glycerogelatin, and the like may be used. When administered intranasally, the pharmaceutical composition may be diluted and administered via nasal spray, which is absorbed into the nasal cavity via a nebulizer or mist system. For the nasal spray, an aerosol agent, etc. may be used as a nasal spray agent or a respiratory preparation.

The content of the active ingredient contained in the pharmaceutical composition may be 0.01 % by weight to 99.9% by weight, 0.01 % by weight to 90% by weight, 0.01 % by weight to 80% by weight, 0.01% by weight to 70% by weight, 0.01% by weight to 60% by weight, 0.01% by weight to 50% by weight, 0.01% by weight to 40% by weight, 0.01% by weight to 30% by weight, 1% by weight to 99.9% by weight, 1% by weight to 90% by weight, 1% by weight to 80% by weight, 1% by weight to 70% by weight, 1% by weight to 60% by weight, 1% by weight to 50% by weight, 1% by weight to 40% by weight, 1% by weight to 30% by weight, 5% by weight to 99.9% by weight, 5% by weight to 90% by weight, 5% by weight to 80% by weight, 5% by weight to 70% by weight, 5% by weight to 60% by weight, 5% by weight to 50% by weight, 5% by weight to 40% by weight, 5% by weight to 30% by weight, 10% by weight to 99.9% by weight, 10% by weight to 90% by weight, 10% by weight to 80% by weight, 10% by weight to 70% by weight, 10% by weight to 60% by weight, 10% by weight to 50% by weight, 10% by weight to 40% by weight, or 10% by weight to 30% by weight, based on the total weight of the pharmaceutical composition. However, the content is not limited thereto, and can be appropriately adjusted depending on the formulation form, administration method, administration purpose, etc. Further, the pharmaceutical composition may further include a pharmaceutically acceptable carrier in addition to the active ingredient. The pharmaceutically acceptable carrier is one that is commonly used for the formulation of drugs containing proteins, nucleic acids, or cells, and may refer to a carrier that does not stimulate living organisms and does not inhibit the biological activity and/or properties of the active ingredient. In one embodiment, the carrier may be one or more kinds selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxy benzoate, propylhydroxybenzoate, talc, magnesium stearate, mineral oil, and the like, but are not limited thereto. The pharmaceutical composition may further include one or more kinds selected from the group consisting of a diluent, an excipient, a lubricant, a wetting agent, a sweetening agent, a flavoring agent, an emulsifying agent, a suspending agent, a preserving agent, and the like, that are commonly used in the preparation of the pharmaceutical composition.

The pharmaceutically effective amount of the pharmaceutical composition may be variously prescribed by various factors such as preparation method, administration mode, excretion rate and reaction sensitivity of the patient, and the composition may be administered or taken once or several times a day, but is not limited thereto and can be administered in various dosages and methods. In one embodiment, a single dose of the pharmaceutical composition may be in the range of 0.0001 to 5000 mg/kg, 0.0001 to 4000 mg/kg, 0.0001 to 3000 mg/kg, 0.0001 to 2500 mg/kg, 0.0001 to 2000 mg/kg, 0.0001 to 1500 mg/kg, 0.0001 to 1000 mg/kg, 0.0001 to 500 mg/kg, 0.0001 to 100 mg/kg, 0.0001 to 50 mg/kg, 0.0001 to 10 mg/kg, 0.0001 to 5 mg/kg, 0.0001 to 1 mg/kg, 0.001 to 5000 mg/kg, 0.001 to 4000 mg/kg, 0.001 to 3000 mg/kg, 0.001 to 2500 mg/kg, 0.001 to 2000 mg/kg, 0.001 to 1500 mg/kg, 0.001 to 1000 mg/kg, 0.001 to 500 mg/kg, 0.001 to 100 mg/kg, 0.001 to 50 mg/kg, 0.001 to 10 mg/kg, 0.001 to 5 mg/kg, 0.001 to 1 mg/kg, 0.01 to 5000 mg/kg, 0.01 to 4000 mg/kg, 0.01 to 3000 mg/kg, 0.01 to 2500 mg/kg, 0.01 to 2000 mg/kg, 0.01 to 1500 mg/kg, 0.01 to 1000 mg/kg, 0.01 to 500 mg/kg, 0.01 to 100 mg/kg, 0.01 to 50 mg/kg, 0.01 to 10 mg/kg, 0.01 to 5 mg/kg, 0.01 to 1 mg/kg, 0.1 to 5000 mg/kg, 0.1 to 4000 mg/kg, 0.1 to 3000 mg/kg, 0.1 to 2500 mg/kg, 0.1 to 2000 mg/kg, 0.1 to 1500 mg/kg, 0.1 to 1000 mg/kg, 0.1 to 500 mg/kg, 0.1 to 100 mg/kg, 0.1 to 50 mg/kg, 0.1 to 10 mg/kg, 0.1 to 5 mg/kg, 0.1 to 1 mg/kg, 1 to 5000 mg/kg, 1 to 4000 mg/kg, 1 to 3000 mg/kg, 1 to 2500 mg/kg, 1 to 2000 mg/kg, 1 to 1500 mg/kg, 1 to 1000 mg/kg, 1 to 500 mg/kg, 1 to 100 mg/kg, 1 to 50 mg/kg, 1 to 10 mg/kg, 1 to 5 mg/kg, 5 to 5000 mg/kg, 5 to 4000 mg/kg, 5 to 3000 mg/kg, 5 to 2500 mg/kg, 5 to 2000 mg/kg, 5 to 1500 mg/kg, 5 to 1000 mg/kg, 5 to 500 mg/kg, 5 to 100 mg/kg, 5 to 50 mg/kg, 5 to 10 mg/kg, 10 to 5000 mg/kg, 10 to 4000 mg/kg, 10 to 3000 mg/kg, 10 to 2500 mg/kg, 10 to 2000 mg/kg, 10 to 1500 mg/kg, 10 to 1000 mg/kg, 10 to 500 mg/kg, 10 to 100 mg/kg, 10 to 50 mg/kg, 20 to 5000 mg/kg, 20 to 4000 mg/kg, 20 to 3000 mg/kg, 20 to 2500 mg/kg, 20 to 2000 mg/kg, 20 to 1500 mg/kg, 20 to 1000 mg/kg, 20 to 500 mg/kg, 20 to 100 mg/kg, 20 to 50 mg/kg, 50 to 5000 mg/kg, 50 to 4000 mg/kg, 50 to 3000 mg/kg, 50 to 2500 mg/kg, 50 to 2000 mg/kg, 50 to 1500 mg/kg, 50 to 1000 mg/kg, 50 to 500 mg/kg, 50 to 100 mg/kg, 100 to 5000 mg/kg, 100 to 4000 mg/kg, 100 to 3000 mg/kg, 100 to 2500 mg/kg, 100 to 2000 mg/kg, 100 to 1500 mg/kg, 100 to 1000 mg/kg, 100 to 500 mg/kg, 200 to 5000 mg/kg, 200 to 4000 mg/kg, 200 to 3000 mg/kg, 200 to 2500 mg/kg, 200 to 2000 mg/kg, 200 to 1500 mg/kg, 200 to 1000 mg/kg, 200 to 500 mg/kg, 500 to 5000 mg/kg, 500 to 4000 mg/kg, 500 to 3000 mg/kg, 500 to 2500 mg/kg, 500 to 2000 mg/kg, 500 to 1500 mg/kg, 500 to 1000 mg/kg, 1500 to 5000 mg/kg, 1500 to 4000 mg/kg, 1500 to 3000 mg/kg, 1500 to 2500 mg/kg, 1500 to 2000 mg/kg, 2000 to 5000 mg/kg, 2000 to 4000 mg/kg, 2000 to 3000 mg/kg, or 2000 to 2500 mg/kg, but is not limited thereto.

### Food composition

Another embodiment provides a food composition for preventing and or treating inflammasome-mediated diseases; and/or Gram-negative bacteria infections or diseases caused by Gram-negative bacteria, for protecting nerve cells and/or regenerating (or producing) nerve cells, comprising the above-mentioned compound.

The food composition may be a health functional food.

The inflammasome-mediated diseases; and/or Gram-negative bacteria infections or diseases caused by Gram-negative bacteria are the same as described above.

The food composition according to one embodiment may mean meat, sausage, bread, chocolate, candies, snacks, confectioneries, pizza, ramen, other noodles, gums, a dairy product including ice cream, various soups, a beverage, tea, a heath drink, an alcoholic beverage, a vitamin complex, a health functional food, and a health food, and the like, and includes all foods that are considered within conventional meaning.

The term `health functional food' is identical to the term 'food for specified health use' (FoSHU), and refers to a food which has high medical and medical effects and is processed to efficiently exhibit a living body-modulating function in addition to nutrient supply. As used herein, the term "functional" means controlling nutrients for the structure or functions of the human body or providing beneficial effects to health purposes, such as physiological effects. The food of the present application may be produced by way of a method commonly used in the art, and raw materials and ingredients typically used in the art may be added thereto for the preparation thereof. Moreover, the dosage form of the food may be produced in various dosage forms without limitation as long as it is a dosage form recognized as a food. The food composition of the present application can be produced in various types of dosage forms, and has an advantage that side effects and the like, which may occur when taking the drug for a long time, are not caused by using a food as a raw material, unlike general drugs, and an advantage of excellent portability. Therefore, the food of the present application can be taken as a supplement to promote the effects of preventing or alleviating inflammasome-mediated inflammatory diseases.

The health food means a food having a health maintenance or promotion effect compared to general foods and a health supplement food means a food for health supplement purposes. The terms health functional food, health food, and health supplement food may be interchangeably used in some cases.

Specifically, the health functional food is a food produced by adding the composition according to one embodiment to food materials such as beverages, teas, spices, gum, and confectioneries, or by encapsulating, powdering, or suspending it, and means that they provides a special health effect during intake. The health functional food has an advantage that side effects, which may occur when taking the drug for a long time, are not caused by using a food as a raw material, unlike general drugs.

Since the food composition according to one embodiment can be taken on a daily basis, it can be expected to have an excellent effect in preventing or alleviating inflammasome-mediated inflammatory diseases, and thus can be very usefully used.

The food composition may further include a physiologically acceptable carrier, but types of the carrier are not particularly limited, and any carrier commonly used in the art may be used. In addition, the food composition may contain additional ingredients that are commonly used in food compositions so as to enhance smell, taste, visuality, etc. For example, the food composition may contain vitamins A, C, D, E, B1, B2, B6, B12, niacin, biotin, folate, pantothenic acid, etc. Furthermore, the food composition may contain minerals such as zinc(Zn), iron(Fe), calcium(Ca), chromium(Cr), magnesium(Mg), manganese(Mn), copper(Cu), and chromium(Cr). Moreover, the food composition may also contain amino acids such as lysine, tryptophane, cysteine, valine, etc.

Further, the food composition may also contain food additives, such as a preservative (potassium sorbate, sodium benzoate, salicylic acid, sodium dehydroacetate, etc.), a disinfectant (bleaching powder, higher bleaching powder, sodium hypochlorite, etc.), an antioxidant (butylhydroxyanisole(BHA), butylhydroxytoluene(BHT), etc.), a colorant (tar color, etc.), a color-developing agent (sodium nitrite, etc.), a bleaching agent (sodium sulfite), a seasoning (monosodium glutamate (MSG), etc.), a sweetener (dulcin, cyclemate, saccharin, sodium, etc.), a flavor (vanillin, lactones, etc.), a swelling agent (alum, potassium D-bitartrate, etc.), a fortifier, an emulsifier, a thickeners (adhesive pastes), a film-forming agent, a gum base agent, an antifoaming agent, a solvent, an enhancer, etc. The additives may be selected according to the type of food and used in an appropriate amount.

The composition according to one embodiment may be added as it is, or may be used in conjunction with other foods or food ingredients, and may be appropriately used according to a conventional method. The mixing amount of active ingredients may be appropriately determined depending on the purpose of use (prophylactic, health or therapeutic treatment). In general, when a food or a beverage is produced, the food composition of the present application may be added in an amount of 50 parts by weight or less, specifically 20 parts by weight or less based on the total weight of the food or the beverage. However, when taken for the purpose of health and hygiene, the food composition may be contained in an amount below the range. In addition, since there is no safety problem, the active ingredients may be used in an amount above the range.

The food composition may be used as, for example, a health beverage composition, and in this case, the health beverage composition may further contain various flavors or natural carbohydrates, as in common beverages. The natural carbohydrates may include monosaccharides such as glucose and fructose; disaccharides such as maltose and sucrose; polysaccharides such as dextrin and cyclodextrin; and sugar alcohols such as xylitol, sorbitol and erythritol. The sweeteners may be natural sweeteners such as thaumatin or a stevia extract; or synthetic sweeteners such as saccharin or aspartame.

In addition, the health beverage composition may contain various nutrients, vitamins, minerals, flavors, colorants, pectic acid and salts thereof, alginic acid and salts thereof, organic acid, protective colloidal thickeners, pH regulators, stabilizers, antiseptics, glycerin, alcohols or carbonating agents. Moreover, the health beverage composition may contain fruit flesh used to prepare natural fruit juices, fruit juice beverages or vegetable beverages. These ingredients may be used individually or in combination. A proportion of the additives is not critical, but is generally selected in the range of 0.01 part by weight to 0.1 part by weight per 100 parts by weight of the health beverage composition of the present application.

The food composition according to one embodiment may contain ingredients in various weight percentages if it can show the effect of preventing or alleviating inflammasome-mediated inflammatory diseases.

### Feed composition, etc.

A still yet another embodiment provides a feed additive (feed additive composition) for preventing and/or alleviating inflammasome-mediated diseases; and/or Gram-negative bacterial infections or diseases caused by Gram-negative bacteria, for protecting nerve cells, and/or for regenerating (or producing) nerve cells, comprising the above-mentioned compound.

The inflammasome-mediated diseases; and/or Gram-negative bacterial infections or diseases caused by Gram-negative bacteria are the same as described above.

Some embodiments provide a feed comprising the above-mentioned feed additive.

For the feed, the antibiotic or combination antibiotic may be prepared separately in a form of a feed additive and mixed with the feed, or it may be prepared by directly adding it during feed preparation.

The compound, antibiotic or combination antibiotic in the feed may be in liquid or dried form, and for example, it may be in a dried powder form. The antibiotic may be contained in an amount of 0.005 to 10% by weight, 0.05 to 10% by weight, 0.1 to 10% by weight, 0.005 to 5% by weight, 0.05 to 5% by weight, 0.1 to 5% by weight, 0.005 to 2% by weight, 0.05 to 2% by weight, or 0.1 to 2% by weight, based on the total weight the feed, but not limited thereto. In addition, the feed may further contain common additives that can increase preservability of the feed in addition to the above compounds, antibiotics, or combination antibiotics.

In the present invention, the feed to which the compound, antibiotic or combination antibiotic can be added may be selected from the group consisting of commercially available feed, or grains, root fruits, food processing by-products, algae, fibers, pharmaceutical by-products, oils and fats, starches, gourds, grain by-products, proteins, inorganic matters, oils and fats, minerals, single cell proteins, zooplankton, leftover food, and the like, but not limited thereto.

Some embodiments provide a food additive or drinking water additive comprising the above-mentioned compound, antibiotic, or combination antibiotic as an active ingredient. By mixing the compound, antibiotic, or combination antibiotic in drinking water and providing it, the number of Gram-negative bacteria in drinking water may be reduced. The Gram-negative bacteria are the same as described above.

Some embodiments provides a disinfectant comprising the above-mentioned compound, antibiotic, or combination antibiotic as an active ingredient. Some embodiments provide a method for disinfection, comprising a step of applying the above-mentioned compound, antibiotic, or combination antibiotic to a subject in need of disinfection. The disinfectant is a generic term for agents to prevent pathogen infection, and may be used for disinfection of general life disinfectants, disinfectants for food and cooking places and facilities, and various kinds of growth and development supplies such as buildings such as poultry farm and pen, livestock bodies, drinking water, litter, egg tray, transport vehicles, tableware, and the like.

Some embodiments provide a detergent comprising the compound, antibiotic or combination antibiotic as an active ingredient. Some embodiments provide a method for cleaning, comprising applying the compound, antibiotic or combination antibiotic into a subject in need of cleaning. As the antibiotic has the antibiotic effect against gram negative bacterium, it may be applied for cleaning (washing) the skin surface or body parts of individuals exposed or likely to be exposed to gram negative bacterium. The gram negative bacterium is same as described above.

### Preparation method

In a final embodiment, a method for preparing the compounds of Chemical Formula 1 is provided.

In one embodiment, the preparation method may comprise a step of introducing phenylboronic acid or a derivative thereof (e.g., phenylboronic acid, Cas No. 98-80-6), 4-methylphenylboronic acid or p-tolylboronic acid (Cas No. 5720-05-8), or (4-methoxyphenyl)boronic acid (Cas No. 5720-07-0) into a compound of the following Chemical Formula 4 to prepare the compound of Chemical Formula 1.

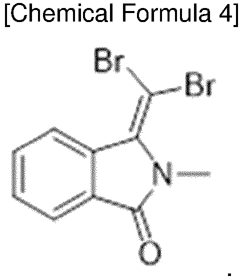

In the compound of Chemical Formula 1,the 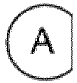 is an unsubstituted aryl, and when R₃ is a C1 alkyl (methyl group), the step of preparing the compound of Chemical Formula 1 may be performed by reacting phenylboronic acid (Cas No. 98-80-6), 4-methylphenylboronic acid or p-tolylboronic acid (Cas No. 5720-05-8), or (4-methoxyphenyl)boronic acid (Cas No. 5720-07-0) with the compound of Chemical Formula 4.

A person having ordinary skill in the art to which the present invention pertains can prepare a compound by various methods based on the structure of Chemical Formula 1, and all of these methods should be construed as being within the scope of the present invention. That is, the compound of Chemical Formula 1 can be prepared within the scope of the present invention by any combination of several synthetic methods described herein or published in the prior art. Therefore, the preparation method according to the present invention is not limited to those presented below.

### [Advantageous Effects]

The present application relates to: a compound of Chemical Formula 1, an isomer thereof or a pharmaceutically acceptable salt thereof; and use thereof for preventing, alleviating and/or treating inflammasome-mediated diseases and/or Gram-negative bacterial infections or diseases caused by Gram-negative bacteria, does not exhibit cell cytotoxicity, and thus is safe, and can effectively inhibit inflammasomes, thereby having the excellent effects of preventing, alleviating and/or treating these diseases

### [BRIEF DESCRIPTION OF THE DRAWINGS]

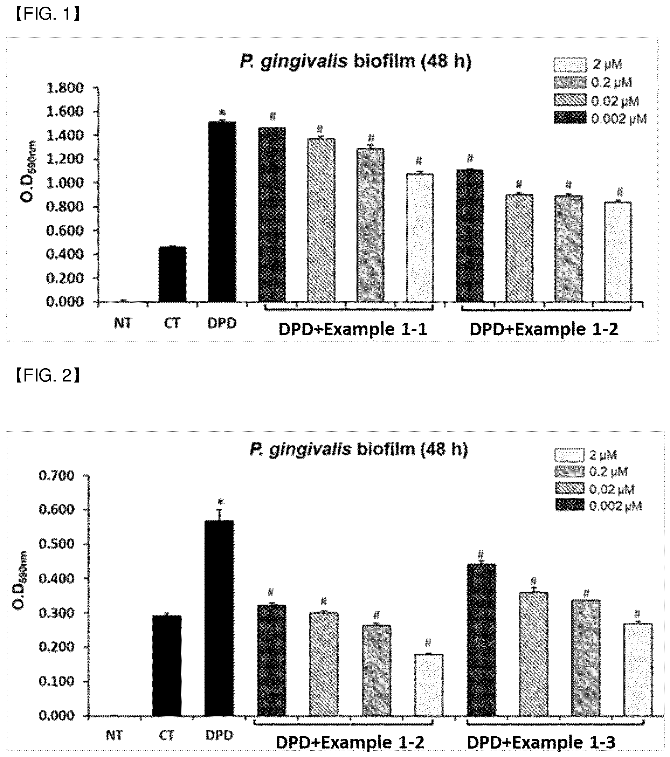

FIGS. 1 and 2 illustrate that the compounds of Examples 1-1 to 1-3 ware treated with different concentrations (2 µM, 0.2 µM, 0.02 µM, 0.002 µM) onto the medium cultured with *P. gingivalis,* and the level of *P. gingivalis* biofilm formation (O.D₅₉₀ₙₘ) was confirmed and shown in graph. "NT" is an untreated group, "DPD" is a group in which the compound DPD was treated onto the culture medium to culture *P. gingivalis,* "CT" is a group in which PBS instead of DPD was treated onto the culture medium to culture *P. gingivalis*, and DPD+Example 1-1, DPD+Example 1-2, and DPD+Example 1-3 are groups in which DPD and the compounds of Examples were treated together.
FIGS. 3a to 3e illustrate that the medium in which the compound of Example 1-2 and the comparative example compound were treated with different concentrations (2 µM, 0.2 µM, 0.02 µM, 0.002 µM) onto the medium cultured with *P*. *gingivalis* and the *P. gingivalis* biofilm formation level (O.D₅₉₀ₙₘ) was confirmed and shown in graph.
FIGS. 4a to 4d are graphs showing the stability of the compound of Example 1-2 and the comparative example compound in human plasma and rat plasma.

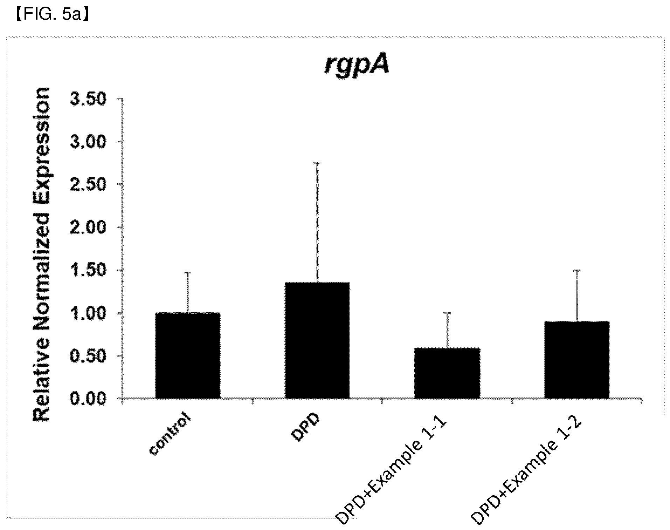

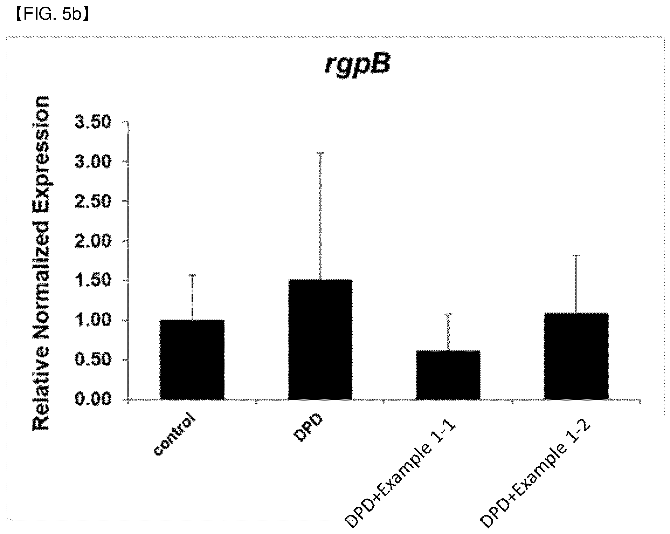

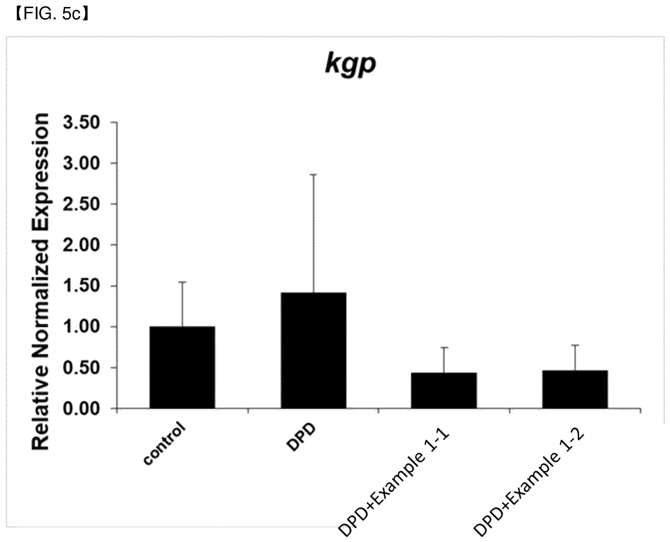

FIGS. 5a to 5c illustrate that the medium in which the compounds of Examples 1-1 to 1-2 were respectively treated onto the medium cultured with *P. gingivalis* and the expression levels of three gingipain proteins (lysine-gingipain(Kgp), arginine-gingipain A(RgpA), and arginine-gingipain B(RgpB)) produced by *P. gingivalis,* which are major virulence factors and proteolytic enzymes are shown in graph. The 16s rRNA gene was used to normalize the expression level of the gingipain gene. "Control" is an untreated group, "DPD" is a group in which the compound DPD was treated onto the culture medium to culture *P. gingivalis*; and DPD+Example 1-1 and DPD+Example 1-2 are groups in which h DPD and the compounds of Examples were treated together.
FIG. 6 illustrates that the compound of Example 1-2 was treated with various concentrations (0.5 µM to 16 µM) onto the mouse bone marrow-derived macrophage and presence/absence of the cytotoxicity was confirmed and shown in graph. "Con" is an untreated control group.
FIGS. 7a to 7d illustrate that as the results of treating Gram-negative bacteria with the example compounds, the *E*. *coli* biofilm was significantly inhibited compared to the positive control (CT) that was not treated with the example compounds. (FIGS. 7a and 7b: results for *E*. *coli,* FIG. 7c: results for *P. aeruginosa,* FIG. 7d: results for A. *baumannii*)
FIG. 8 shows that as a result of treating *E*. *coli* with both the example compound and an antibiotic, the minimum growth inhibitory concentration of the antibiotic was significantly reduced compared to the case where the antibiotic was treated alone.
FIGS. 9 and 10 are schematic diagrams showing that the compound of the present application inhibits the biofilm formation of Gram-negative bacteria.
FIG. 11 shows that inflammation was induced in nerve cells by the stimulation source of *P. gingivalis* infection, resulting in cell damage, but the nerve cells were protected by treatment with the example compound.
FIGS. 12 and 13 are graphs showing that gene expression of inflammatory cytokines in nerve cells was increased by the stimulation source of *P. gingivalis* infection, but neuroinflammation was alleviated by treatment with the example compounds.
FIG. 14 is a graph showing that the example compound do not exhibit toxicity to nerve cells.
FIG. 15 is a graph showing the concentration in plasma over time when the example compound was intravenously injected into a male rat to collect a blood sample.
FIG. 16 is a graph showing the intracerebral concentration over time when the example compound was intravenously injected into a male rat to collect a brain sample, which shows that the example compound passes through the blood and then the blood-brain barrier to reach the brain, and therefore can act on the intracerebral lesion, which is the target site, thereby treating neurodegenerative diseases.
FIG. 17 is a graph showing that the example compound was treated onto the mouse bone marrow-derived macrophages, and changes in the production of inflammatory cytokines (IL-1β) in the macrophages were measured, and as a result, the inflammatory response induced in the macrophages was suppressed by the example compound.
FIG. 18 is a graph showing that the example compound does not exhibit cytotoxicity in mouse bone marrow-derived macrophages.
FIG. 19 is a graph showing that the example compound does not exhibit cytotoxicity in human monocyte cell lines.

### [DETAILED DESCRIPTION OF THE EMBODIMENTS]

Hereinafter, the present invention will be described in more detail by way of Examples. However, these Examples are given for illustrative purposes only, and the scope of the invention is not intended to be limited to or by these Examples.

### Example 1. Preparation of Compound

### Example 1-1. Preparation of 3-(diphenylmethylene)-2-methylisoindolin-1-one

Compound of Example 1-1 (3-(diphenylmethylene)-2-methylisoindolin-1-one (CAS No. 92172-54-8 )) was prepared by carrying out the following steps.

### Step 1: Preparation of Compound A

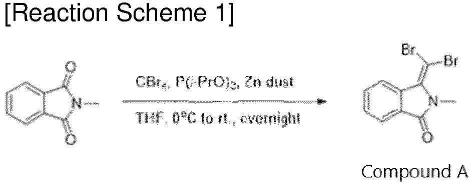

In 10 mL of THF (Cas No. 109-99-9, Sigma Aldrich) containing Zn dust (Cas No. 7440-66-6, Sigma Aldrich) (490 mg, 7.50 mmol), triisopropylphosphite (P(i-PrO)₃, Cas No. 116-17-6, Sigma Aldrich) (2.7 ml, 11.00 mmol) was cooled to 0°C under Argon (Cas No. 7440-37-1, Sung Kwang Specialty Gas). A solution of CBr₄ (Cas No. 558-13-4, TCI) (2.49 g, 7.50 mmol) in THF was added slowly to the solution of P(i-PrO)₃ and the reaction mixture was stirred until the color changed to yellow. Then, a solution of N-methylphthalimide (Cas No. 550-44-7, Sigma Aldrich) (806 mg, 5.00 mmol) in THF was added thereto. The reaction mixture was stirred at room temperature for 12 hours. After sat. aq NaHCOs solution (Cas No. 144-55-8, Samchun Chemicals) (2.5 mL) was added and quenched, the phases were then isolated and a crude product was extracted from an aqueous layer with ether. The residue was dissolved in ether and filtered through a Celite pad. The resulting filtrate was concentrated by rotary evaporation and purified by column chromatography (50% dichloromethane (DCM) in hexane) to obtain Compound A.

### Step 2: Preparation of the compound of Example 1-1

Under an argon atmosphere, Compound A (158 mg, 0.5 mmol) prepared in step 1, Na₂CO₃ (212 mg, 2 mmol), Pd(OAC)₂ (Cas No. 3375-31-3, TCI) (22 mg, 0.1 mmol), PPhs (Cas No. 603-35-0, TCI) (52 mg, 0.2 mmol), H₂O (2 ml) and THF (8 ml) were added to a Schlenk tube. Phenylboronic acid (Cas No. 98-80-6, TCI) (1.2 mmol) was added to the mixture under stirring, and then the mixture was stirred at room temperature for 24 hours. The reaction mixture was extracted with DCM (Cas No. 75-09-2, Sigma Aldrich) (10 mL), washed with brine, dried with MgSO₄ (Cas No. 10034-99-8, Samchun Chemicals), and filtered. The crude product was purified by flash chromatography on silica gel to obtain the compound of Example 1-1.

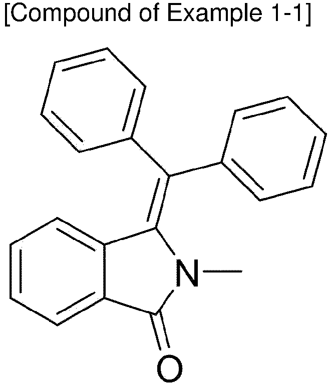

### Example 1-2. Preparation of 3-(di-p-tolylmethylene)-2-methylisoindolin-1-one

The compound of Example 1-2 (3-(di-p-tolylmethylene)-2-methylisoindolin-1-one) was prepared by carrying out the following steps.

### Step 1: Preparation of Compound A

Compound A was prepared in the same manner as in step 1 of Example 1-1.

### Step 2: Preparation of Compound of Example 1-2

Under an argon atmosphere, Compound A (158 mg, 0.5 mmol) prepared in step 1, Na₂CO₃ (212 mg, 2 mmol), Pd(OAC)₂ (22mg, 0.1 mmol), PPhs (52 mg, 0.2 mmol), H₂O (2ml) and THF (8 ml) were added to a Schlenk tube. 4-Methylphenylboronic acid or p-tolylboronic acid (Cas No. 5720-05-8, TCI) (1.2 mmol) was added to the mixture under stirring, and then the mixture was stirred at room temperature for 24 hours. The reaction mixture was extracted with DCM (10 mL), washed with brine, dried over MgSO₄ and filtered. The crude product was purified by flash chromatography on silica gel to obtain the compound of Examples 1-2.

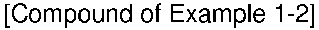

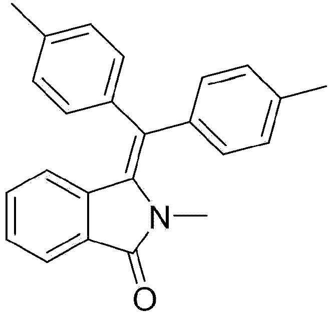

### Example 1-3. Preparation of 3-(bis(4-methoxyphenyl)methylene)-2-methylisoindolin-1-one

Compound of Example 1-3 (3-(bis(4-methoxyphenyl)methylene)-2-methylisoindolin-1-one) was prepared by carrying out the following steps.

### Step 1: Preparation of Compound A

Compound A was prepared in the same manner as in step 1 of Example 1-1.

### Step 2: Preparation of Compound of Examples 1-3

Under an argon atmosphere, Compound A (158 mg, 0.5 mmol) prepared in step 1, Na₂CO₃ (212 mg, 2 mmol), Pd(OAC)₂ (22mg, 0.1 mmol), PPhs (52 mg, 0.2 mmol), H₂O (2ml) and THF (8 ml) were added to a Schlenk tube. 4-(Methoxyphenyl)boronic acid (Cas No. 5720-07-0, TCI) (1.2 mmol) was added to the mixture under stirring, and then the mixture was stirred at room temperature for 24 hours. The reaction mixture was extracted with DCM (10 mL), washed with brine, dried over MgSO₄ and filtered. The crude product was purified by flash chromatography on silica gel to obtain the Compound of Examples 1-3.

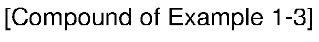

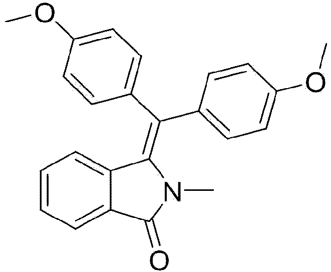

### Preparation of Comparative Example Compound DPD

Compound DPD (4,5-dihydroxy-2,3-pentanedione, CAS NO. 142937-55-1) used in Experimental Example described hereinafter was prepared by carrying out the following steps 1 to 5 (See Molecules. 2018 Oct; 23(10): 2545).

Step 1: 1-Propynylmagnesium bromide (0.5 M in THF, 1.3 eq) was slowly added to a solution of anhydrous (t-butyldimethylsilyloxy)acetaldehyde (1.0 eq) in THF at 0°C. The mixture was stirred at room temperature for 3 hours. The reaction solution was concentrated by rotary evaporation, and then quenched by addition of cooled saturated NH₄Cl solution. The reaction mixture was then extracted with Et₂O, washed with brine, dried over MgSO₄ and filtered. Yellow oily substance 1 was obtained.

Step 2: The obtained substance 1 (1.0 eq) was dissolved in MeOH, Dowex50WX8 100-200 mesh (100 mg/1 mL) was added thereto and stirred at room temperature for 12 hours. The product was filtered to obtain a substance 2.

Step 3: Cyclohexanone dimethyl ketal (3.0 eq) and catalyst p-TSA were added to the obtained substance 2 (1.0 eq), and stirred at room temperature for 12 hours. The reaction solution was concentrated by rotary evaporation, then dissolved in Et₂O and washed with NaHCOs, dried over MgSO₄ and filtered to obtain a substance 3.

Step 4: The obtained substance 3 was added to a 1:1:1 CHCl₃/ACN/H₂O solution, and NaIO₄ (4.4 eq) and RuO₂_H₂O (2.5% mol) were added thereto, and stirred at room temperature for 12 hours. The reaction solution was concentrated by rotary evaporation, then extracted with DCM and dried over MgSO₄ to give a substance 4.

Step 5: The obtained substance 4 was added to MeOH, and Dowex 50WX8 resin was added thereto, and stirred at room temperature for 12 hours. The reaction mixture was filtered to obtain a compound DPD.

### Example 2. Inhibitory effect on biofilm formation in P. gingivalis

The inhibitory effect of the compounds of Examples 1-1 to 1-3 on biofilm formation of *P. gingivalis* (*Porphyromonas gingivalis*) was confirmed.

The compounds of Examples 1-1 to 1-3 were respectively treated onto the medium cultured with *P. gingivalis* (provided and used by the Korean Collection for Oral Microbiology(KCOM)) and the *P. gingivalis* biofilm formation level was confirmed.

Sterilized cover glass slips (round, 12 mm radius) were placed individually at a bottom of each well of a cell culture plate (24-well plate) using forceps, and DPD (4,5-dihydroxy-2,3-pentanedione, CAS NO. 142937-55-1), which is a precursor of the interbacterial signaling substance AI-2 (Autoinducer-2), was added to a concentration of 10 µM to the medium (BHI medium + supplements) cultured with *P. gingivalis* (BHI, a component of the medium, is Brain Heart Infusion Medium, and hemin (10 µg/ml) and vitamin K (0.2 µg/ml) were used as supplements). It is known that as bacteria use DPD for quorum sensing, bacterial biofilm formation increases. The example compounds (compounds of Examples 1-1 to 1-3) were respectively diluted in a medium containing DPD, and prepared so that the final concentrations thereof were 0.002 µM, 0.02 µM, 0.2 µM, and 2 µM, respectively.

The prepared culture medium was dispensed into a cell culture plate in which glass slips were placed, and the bacteria were inoculated to a concentration of 2 × 10⁸ cells/ml. In the case of the positive control (DPD) which was not treated with the example compound, DPD was added to the medium (BHI medium + supplement) and then the same number of bacteria was inoculated. In the case of the negative control (CT) which was not treated with both the example compound and DPD, PBS(Phosphate Buffered Saline) instead of DPD was added in the same amount to the culture medium (BHI medium + supplement). The completed cell culture plate was maintained at 37°C under anaerobic conditions (10% H₂, 10% CO₂, and 80% N₂) for 48 hours to culture bacteria.

Biofilms in which glass slips by bacteria cultured for each group were formed on glass slips was stained with 1% crystal violet solution for 15 minutes, and then washed three times with PBS (Phosphate buffered saline) solution, and decolorized with acetone-alcohol (20:80, vol/vol). After the decolorizing solution containing crystal violet was put in 200 µl increments in a microplate for each group, the absorbance (OD₅₉₀ₙₘ) was measured using an absorbance microplate reader (Epoch2, Bio-Tek, USA), and the results were compared and analyzed by group. The results are shown in FIGS. 1 and 2.

As shown in FIGS. 1 and 2, no biofilm formation was observed in the medium (NT) which was not cultured with *P. gingivalis.* In the case of culture medium (DPD) in which DPD was added to the medium to culture *P. gingivalis,* the biofilm formation of *P. gingivalis* was significantly increased through quorum sensing action as compared to the medium (CT) in which PBS was added to the medium to culture *P. gingivalis.* In the case of a medium (DPD+Example 1-1, DPD+Example 1-2, DPD+Example 1-3) in which DPD and the example compounds were added together to culture *P. gingivalis,* it was confirmed that the biofilm formation of *P. gingivalis* increased by DPD was inhibited in a concentration-dependent manner of the example compound.

### Comparative experiment

To demonstrate that the compounds of Examples 1-1 to 1-3 have a superior effect to the comparative compounds, the following experiment was carried out. As the comparative example compound, the compound ((Z)-3-(bromo(phenyl)methylene)isobenzofuran-1(3H)-one) of <Example 1> of Korean Unexamined Patent Publication No. 10-2019-0130983A was used.

### 1. Inhibitory effect on P. gingivalis biofilm formation

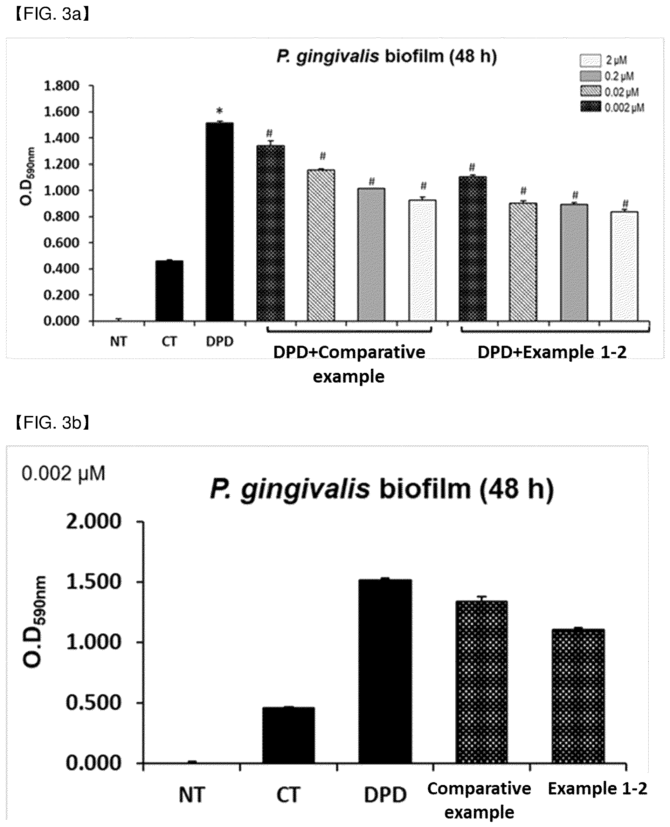

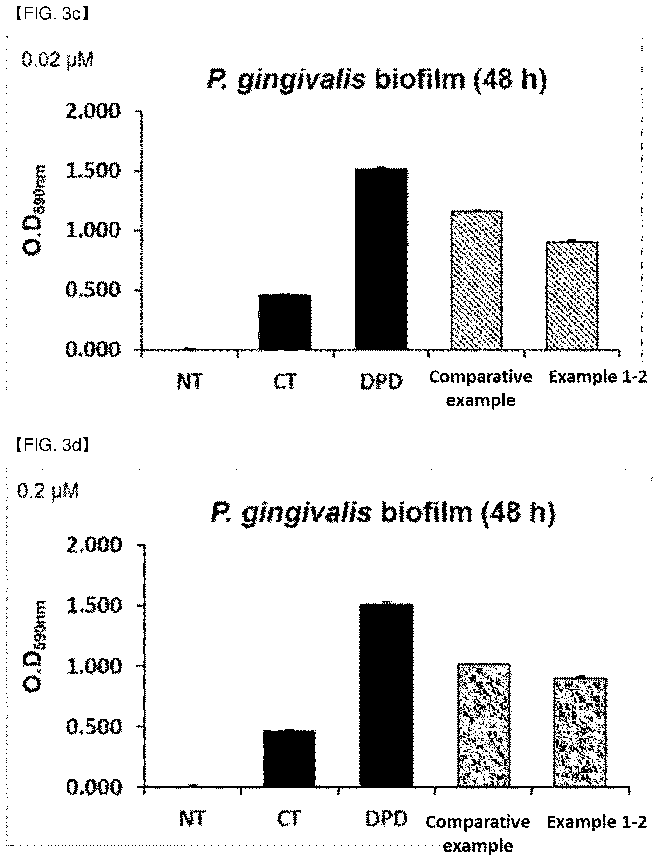

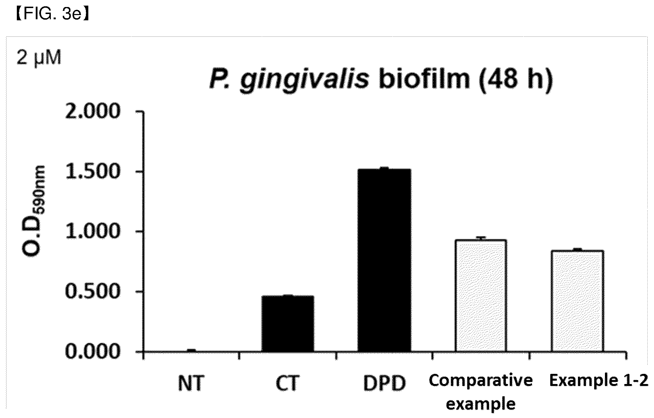

The inhibitory effect of the compound of Example 1-2 and the comparative example compound on *P. gingivalis* biofilm formation was confirmed. The experimental process was carried out in the same manner as in Experimental Example 1, and the experimental results are shown in FIGS. 3a to 3e.

As can be seen in FIGS. 3a to 3e, no biofilm formation was observed in the medium (NT) which was not cultured with *P. gingivalis.* In the case of culture medium (DPD) in which DPD was added to the medium to culture *P. gingivalis,* the biofilm formation of *P. gingivalis* was significantly increased through quorum sensing action as compared to the medium(CT) in which PBS was added to the medium to culture *P*. *gingivalis.* In the case of a medium (DPD+Comparative Example, DPD+Example 1-2) in which DPD and the example compounds were added together to culture *P. gingivalis,* it was confirmed that the biofilm formation of *P. gingivalis* increased by DPD was inhibited in a concentration-dependent manner of the compound. When comparing the ability to inhibit biofilm formation of *P. gingivalis* at the same concentration, the compound of Example 1-2 was confirmed to have a better ability to inhibit biofilm formation than the comparative example compound.

### 2. Plasma stability

The plasma stability of the compound of Example 1-2 and Comparative Example in human plasma and rat plasma was compared.

### 1) Sample preparation

The compounds of Examples 1-2 and Comparative Example, and the substance used as the positive control (Eucatropine, Loterpresnol) were dissolved in DMSO to prepare a stock solution having a concentration of 10 mM. The stock solution of each test substance was then diluted to prepare 0.2mM and 1mM working solutions and used for the test (For the comparative example compound, 70% acetonitrile was used as a solvent, and for the compound of Example 1-2, 50% acetonitrile was used as a solvent).

### 2) Test method

The positive control and the standard solution of the test substance were placed in tubes containing two types of plasma (human and rat) at concentrations of 1 µM and 5 µM, respectively, and then incubated at 37°C for a prescribed time (0, 30, 60, 120, 240 minutes) while shaking and mixing. At each time point, the mixture sample was dispensed in 100 µl increments into a cell culture plate (96-well plate) containing 400 µl of quench reagent to terminate the test, After centrifugation (5000 × g, 10 min), 100 µl of the supernatant was taken out, mixed with 200 µl of ultrapure water, and the solution was then injected into a LC-MS/MS system to analyze the drug in each time zone. Thereby, the plasma stability of the compounds of Comparative Example and Example 1-2 was evaluated.

### 3) Test results

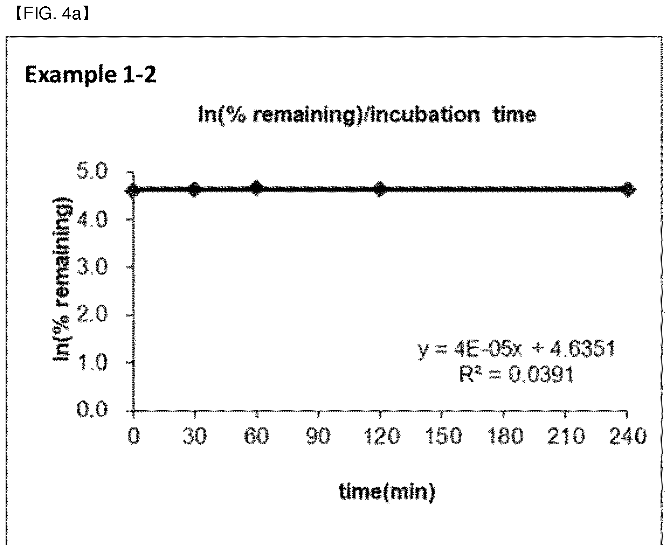

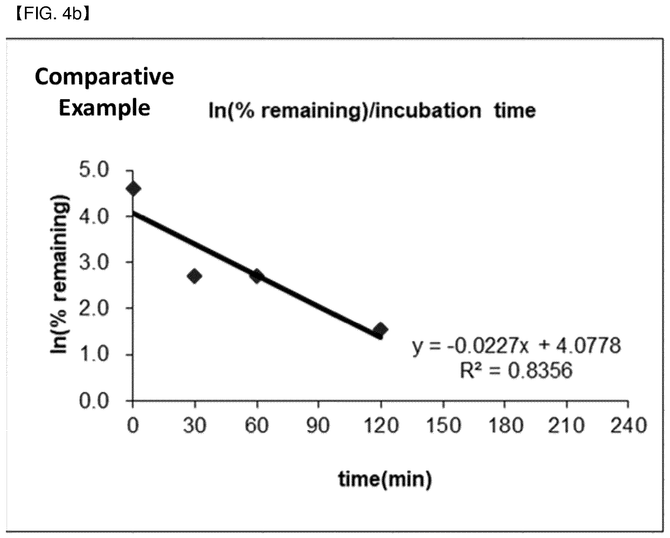

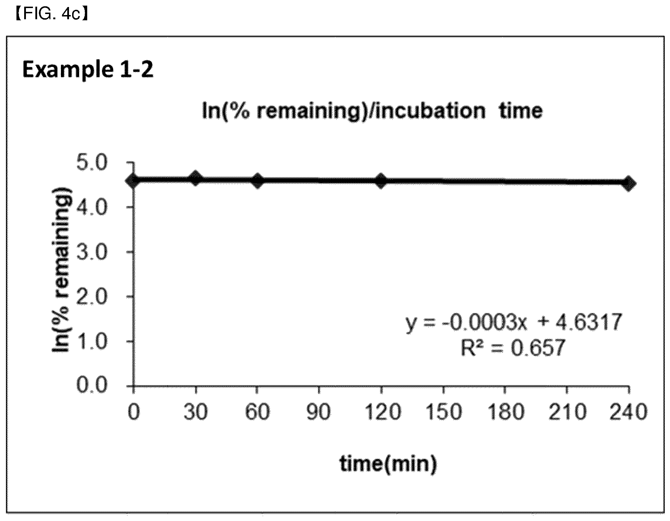

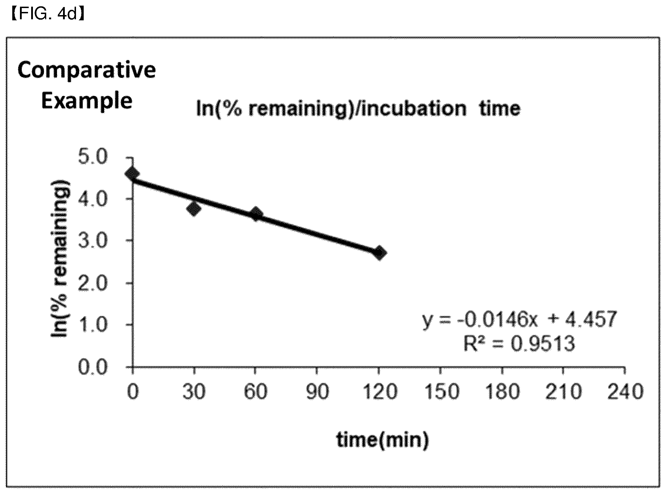

The results showing the stability in human plasma are shown in FIG. 4a (compound of Example 1-2) and FIG. 4b (compound of Comparative Example), and the results showing the stability in rat plasma are shown in FIG. 4c (compound of Example 1-2) and FIG. 4d (compound of Comparative Example).

As can be seen in FIGS. 4a to 4d, it was confirmed that for the comparative example compound, the amount of the compound remaining in the plasma decreased over time in human plasma and rat plasma (FIGS. 4b and 4d), but the compound of Example 1-2 remained stable in plasma (FIGS. 4a and 4c).

### Example 3. Inhibitory effect on gingipain expression of P. gingivalis

The compounds of Examples were respectively treated onto the medium cultured with *P. gingivalis,* and the expression levels of three gingipain proteins (lysine-gingipain(Kgp), arginine-gingipain A(RgpA), and arginine-gingipain B(RgpB)) produced by *P. gingivalis,* which are major virulence factors and proteolytic enzymes, were confirmed.

The culture medium containing 10 µM of DPD (4,5-dihydroxy-2,3-pentane dione), which is a precursor of the bacterial signal substance (Autoinducer-2), and the culture medium containing 2 µM of the example compound or not were respectively dispensed into a cell culture plate (48 well plate), and then *P. gingivalis* (2×10⁸ cell/ml) were inoculated for each group. The culture medium cultured with bacteria was cultured at 37°C under anaerobic conditions (10% H₂, 10% CO₂, and 80% N₂) for 48 hours.

After 48 hours, each culture medium was centrifuged at 13,000 rpm for 1 minute to remove the supernatant, and total RNA was extracted using easy-BLUETM Total RNA Extraction kit (iNtRON Biotechnology, Sungnam, Korea) to synthesize cDNA, and then the expression of the gingipain gene was confirmed by real-time PCR method. cDNA was synthesized using AccuPower^{®} CycleScript RT PreMix dN6 (Bioneer, Daejeon, Korea), and real-time (quantitative) reverse transcription PCR (RT-qPCR) was carried out using Power SYBR^{™} Green PCR Master Mix (Applied Biosystems, Foster City, CA, USA).

The primer sequences used for amplification are shown in Table 1 below. RT-qPCR amplification conditions were as follows: after enzyme activation at 95°C for 3 minutes, denaturation at 95°C for 10 seconds and primer annealing at 60°C for 30 seconds were repeated 40 times, and analysis was performed using a Bio-Rad CFX Connect Real-Time PCR Detection System (Bio-Rad, Hercules, CA, USA). To normalize the expression level of the gingipain gene, the 16s rRNA gene was used, and the gene expression level was calculated using the Comparative Ct Method (ΔΔCt), which is shown in FIGS. 5a to 5c.

**[Table 1]**

| Gene | Primer sequence |
|---|---|
| Lysine-gingipain (Kgp) | fwd: 5'-CAACCAAAGCCAAGAAGA-3' (SEQ ID NO: 1) |
| | rev: 5'-CGAAGCTGAAGTAGGAAC-3' (SEQ ID NO: 2) |
| Arginine-gingipain A (RgpA) | fwd: 5'-GCTCTTTCCATAAACGTAAG-3' (SEQ ID NO: 3) |
| | rev: 5'-GACACTCGTGAGATGAAG-3' (SEQ ID NO: 4) |
| Arginine-gingipain B (RgpB) | fwd: 5'-CTTCCACTTTCACATCCTTTA-3' (SEQ ID NO: 5) |
| | rev: 5'-CTGACGATGGTGATATGG-3' (SEQ ID NO: 6) |
| 16s rRNA | fwd: 5'-ACGAGTATTGCATTGAATG-3' (SEQ ID NO: 7) |
| | rev: 5'-ACCCTTTAAACCCAATAAATC-3' (SEQ ID NO: 8) |

As shown in FIGS. 5a to 5c, when compared with an untreated group (control group), in the case of the group treated with DPD which is a precursor of AI-2 (DPD), *P. gingivalis* recognized the quorum sensing signal molecule, and all the expression levels of three gingipain genes (rgpA, rgpB, kgp) increased more than in the control group. In the case of the group treated with DPD and the example compounds together (DPD + Example 1-1, DPD + Example 1-2), it was confirmed that the expression levels of three gingipain genes increased by DPD were reduced.

### Example 4. Cytotoxicity evaluation

It was evaluated whether the example compound exhibited cytotoxicity in mouse bone marrow-derived macrophages.

Bone marrow was isolated from the femur and tibia of 8-12 week old C57BU6J mice (available from Daehan Biolink) using a 26 g syringe, and the isolated bone marrow was differentiated into macrophages by culturing a medium containing IMDM media + 30% L929 cell supernatant + 10% fetal bovine serum + 1% pencillin/streptomycin + 1% non-essential amino acid + 1% sodium pyruvate in a 150π cell culture plate for 6 days, and used for the experiment.

The differentiated macrophages were seeded in 200 µl increments in a 48-well plate at a concentration of 1×10⁶ cell/ml, and stabilized at 37°C and 5% CO₂ for 12 hours. After the supernatant of the cultured cells was removed, the compound of Example 2 was diluted to concentrations of 0.5, 1, 2, 4, 8 and 16 µM in a serum free medium, treated in 200 µl increments, and cultured at 37°C and 5% CO₂ for 24 hours.

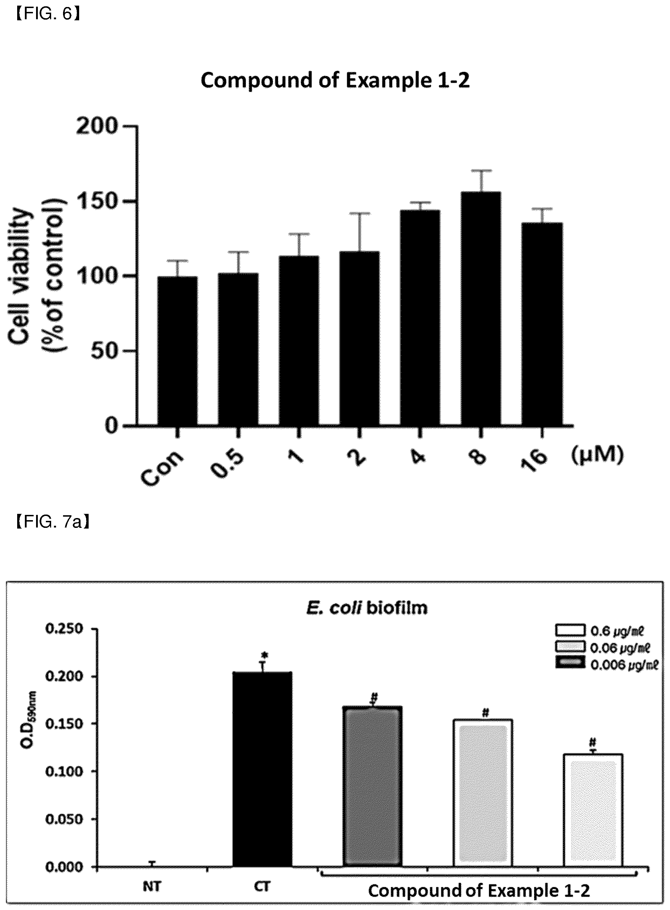

Then, the supernatant of the cultured cells was removed, treated with 200 µl of MTT solution having a concentration of 400 µg/ml, and then reacted at 7°C and 5% CO₂ for 4 hours. Then, the reaction solution was removed, 200 µl of DMSO was added, and the absorbance was measured at 570 nm. At this time, the viability of cells treated with the sample was calculated based on the viability of untreated cells considered as 100%, and is shown in FIG. 6.

As can be seen in FIG. 6, it was confirmed that the compound of Example 1-2 did not exhibit cytotoxicity at a concentration of 16 µM or less in mouse bone marrow-derived macrophages.

### Example 5. Inhibitory effect on biofilm formation in E. coli, P. aeruginosa, and A. baumannii

### 1) Inhibitory effect on E. coli biofilm formation

The inhibitory effect of the compound of Example 1-2 on biofilm formation in *E. coli* (KCTC 2571(ATCC 8739); available from the Korean Collection for Oral Microbiology (KCOM)) was confirmed.

Sterilized cover glass slips (round, 12 mm radius) were placed individually at a bottom of each well of a cell culture plate (24-well plate) using forceps, and the compound of Example 1-2 was diluted in the medium (LB medium) to be cultured with bacteria so that the final concentrations were 0.006 µg/ml, 0.06 µg/ml, 0.6 µg/ml, 1.2 µg/ml, 2.4 µg/ml, and 4.8 µg/ml.

The prepared culture medium was dispensed into a cell culture plate in which glass slips were placed, and the bacteria were inoculated to a concentration of 2 × 10⁷ cells/ml. In the case of the positive control (DPD) which was not treated with the compound, the same number of bacteria was inoculated into medium (LB) which did not contain the above compound. In the case of the negative control (CT) which was not treated with both the compound and bacteria, only the medium (LB medium) was treated alone. The completed cell culture plate was maintained at 37°C under anaerobic conditions (10% H₂, 10% CO₂, and 80% N₂) for 48 hours to culture the bacteria.

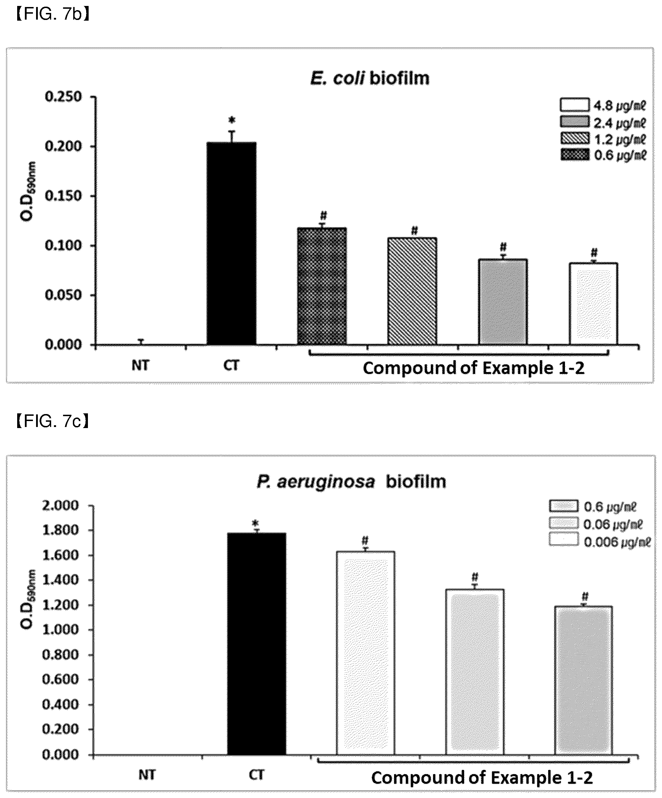

The biofilm in which bacteria cultured for each group were formed on glass slips was stained with 1% crystal violet solution for 15 minutes, and then washed three times with PBS (phosphate buffered saline) solution, and decolorized with acetone-alcohol (20:80, vol/vol). After the decolorizing solution containing crystal violet was put in 200 µl increments in a 96-well microplate for each group, the absorbance (OD₅₉₀ₙₘ) was measured using an absorbance microplate reader (Epoch2, Bio-Tek, USA), and the biofilm formation levels were compared. The results are shown in FIGS. 7a and 7b.

### 2) Inhibitory effect on biofilm formation in P. aeruginosa and A. baumannii

The inhibitory effect of the compound of Example 1-2 on biofilm formation in *P. aeruginosa* (available from the Korean Collection for Oral Microbiology (KCOM)) and A. *baumannii* (available from the Korea Culture Center of Microorganisms (KCCM)) was confirmed.

Sterilized cover glass slips (round, 12 mm radius) were placed individually at a bottom of each well of a cell culture plate (24-well plate) using forceps, and the compound of Example 1-2 was diluted in the medium (*P*. *aeruginosa:* LB medium, A. *baumannii:* NB medium) cultured with bacteria so that the final concentrations were 0.006 µg/ml, 0.06 µg/ml, and 0.6 µg/ml.

The prepared culture medium was dispensed into a cell culture plate in which glass slips were placed, and the respective bacteria were inoculated to a concentration of 2 × 10⁷ cells/ml. In the case of the positive control (CT) which was not treated with the compound, only the same number of bacteria was inoculated into the medium (LB or NB) which did not contain the above compound. In the case of the negative cotrol (NT) which was not treated with both the compound and bacteria, only the medium (LB or NB) was treated alone. The completed cell culture plate was maintained at 37°C under anaerobic conditions (10% H₂, 10% CO₂, and 80% N₂) for 48 hours to culture the bacteria.

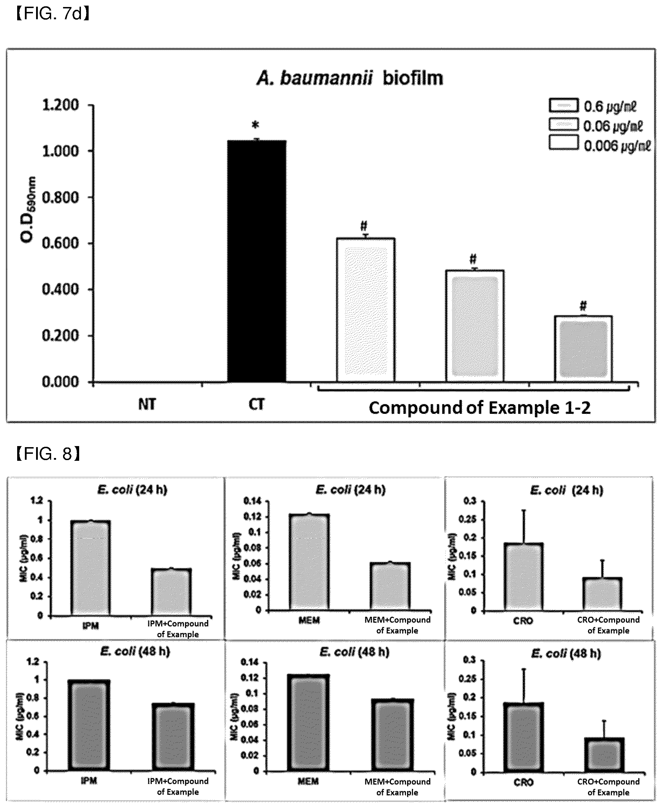

The biofilm in which bacteria cultured for each group were formed on glass slips was stained with 1% crystal violet solution for 15 minutes, and then washed three times with PBS (phosphate buffered saline) solution, and decolorized with acetone-alcohol (20:80, vol/vol). After the decolorizing solution containing crystal violet was put in 200 µl increments in a 96-well microplate for each group, the absorbance (OD₅₉₀ₙₘ) was measured using an absorbance microplate reader (Epoch2, Bio-Tek, USA), and the biofilm formation level was compared. The results are shown in FIGS. 7c and 7d.

As can be seen in FIGS. 7a to 7d, it was confirmed that after 48 hours of incubation, the biofilm of respective bacteria (*E*. *coli, P. aeruginosa,* and *A. baumannii*) increased in the control group (CT), but in the group treated with the compound of Example 1-2, the biofilm formation was significantly suppressed in a concentration-dependent manner.

### Example 6. Combination Effect of antibiotic on E.coli

After 100 µl of *E*. *coli* culture medium (LB) was dispensed into the cell culture plate (96-well plate), three antibiotics (Imipenem (IPM, Sigma-Aldrich), Meropenem (MEM, Sigma-Aldrich), and Ceftriaxone (CRO, Sigma-Aldrich)) as a control group, were administered at a final concentration of 0.0312 µml to 32 µg/ml, and serially diluted. In order to confirm whether the efficacy of the antibiotics increases due to treatment with the compounds of Examples 1-2, in the experimental group, the compound of Example 1-2 was mixed so that each antibiotic dilution solution contained 1 µg/ml. The final volume of the control and experimental groups was 200 µl, and *E*. *coli* was inoculated into each well at 1×10⁵ cells/ml, and then cultured at 37°C for 24 to 48 hours. After the culture was completed, the absorbance (OD₆₀₀ₙₘ) of each well was measured using an absorption microplate reader (Epoch2, Bio-Tek, USA), the minimum growth inhibitory concentrations of the control group and the experimental group were compared, and the results are shown in FIG. 8.

As can be seen in FIG. 8, it was confirmed that the minimum growth inhibitory concentration of three antibiotics (Imipenem (IPM), Meropenem (MEM), and Ceftriaxone (CRO)) against *E*. *coli* was reduced by 20% to 50% or more due to the example compounds. Through these results, it was confirmed that when the antibiotic was treated in combination with the example compounds of the present invention, the efficacy of existing antibiotics was improved more than when the antibiotic was treated alone.

### Example 7. Confirmation of inflammation inhibition and protective effect on nerve cell

Human neuroblastoma (SH-SY5Y cell) (available from Korea Cell Line Bank) were treated at 3×10⁶ cells/well on a cell culture plate (6-well plate), and then cultured in an incubator at 37°C and 5% CO₂ for 24 hours. Only the nerve cells attached to the cell culture plate were treated with 3 ml of culture medium in the negative control, and *P. gingivalis* (obtained and used from the Korean Collection for Oral Microbiology (KCOM)) was inoculated at an infection rate of 500 (MOI 500) in the positive control. In order to confirm the inflammation- alleviating effect of the example compound in nerve cells, the same number of *P. gingivalis* was inoculated in the experimental group, and then the compound of Example 1-2 was diluted and treated onto the medium to 1 µg/ml (2.95 µM).

The completed cell culture plates for each group were cultured in an incubator at 37°C and 5% CO₂ for 24 hours. After the culture was completed, the morphological changes of nerve cells due to the inflammatory response were confirmed through light microscopy analysis for each group, and the results are shown in FIG. 11.

As can be seen in FIG. 11, it was confirmed that in the nerve cells where inflammation was induced by the stimulation source of *P. gingivalis* infection, cell damage such as deformation of cell morphology was confirmed, but the nerve cells were protected by treatment with the compound of the example compounds.

### Real-time PCR analysis

After the optical microscopy analysis was completed, the supernatant was removed from the cell culture plate, and the remaining cells were analyzed for inflammatory cytokine levels in nerve cells for each group through real-time PCR analysis. Each group was treated with 1 ml of a reagent from easy-BLUETM Total RNA Extraction kit (iNtRON Biotechnology, Sungnam, Korea) to lyse nerve cells, and then total RNA was extracted from the lysate to synthesize cDNA. Inflammatory cytokine gene expression was confirmed using real-time PCR method. cDNA was synthesized with AccuPower^{®} CycleScript RT PreMix dT20 (Bioneer, Daejeon, Korea), and real-time (quantitative) reverse transcription PCR (RT-qPCR) was performed using Power SYBR^{™} Green PCR Master Mix (Applied Biosystems, Foster City, CA, USA).

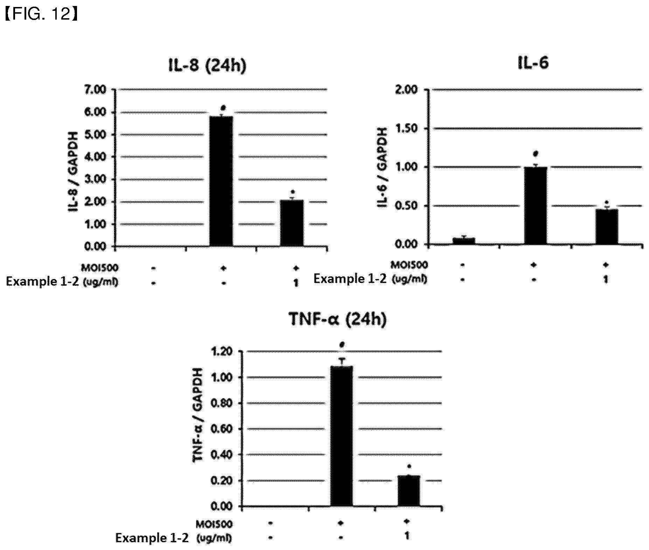

The primer sequences used are shown in Table 2 below, and the amplification conditions were as follows. after enzyme activation at 95°C for 10 minutes, denaturation at 95°C for 10 seconds and primer annealing at 60°C for 30 seconds were repeated 40 times. The device used was the Bio-Rad CFX Connect Real-Time PCR Detection System (Bio-Rad, Hercules, CA, USA). Based on the expression level of the housekeeping gene GAPDH (Glyceraldehyde-3-phosphate dehydrogenase), the relative expression level of inflammatory cytokine genes was calculated using the Comparative Ct Method (ΔΔCt), and the results are shown in FIG. 12.

**[Table 2]**

| Gene | Primer sequence |
|---|---|
| TNF-α | fwd: 5'-CAGAGGGAAGAGTTCCCCAG-3' (SEQ ID NO: 9) |
| | rev: 5'-CCTCAGCTTGAGGGTTTGCTAC-3' (SEQ ID NO: 10) |
| IL-6 | fwd: 5'-GTGAGGAACAAGCCAGAGC-3' (SEQ ID NO: 11) |
| | rev: 5'-TACATTTGCCGAAGAGCC-3' (SEQ ID NO: 12) |
| IL-8 | fwd: 5'-TTTTGCCAAGGAGTGCTAAAGA-3' (SEQ ID NO: 13) |
| | rev: 5'-AACCCTCTGCACCCAGTTTTC-3' (SEQ ID NO: 14) |
| GAPDH | fwd: 5'-GAAGGTGAAGGTCGGAGTC-3' (SEQ ID NO: 15) |
| | rev: 5'-AGATGGTGATGGGATTTC-3' (SEQ ID NO: 16) |

### ELISA analysis

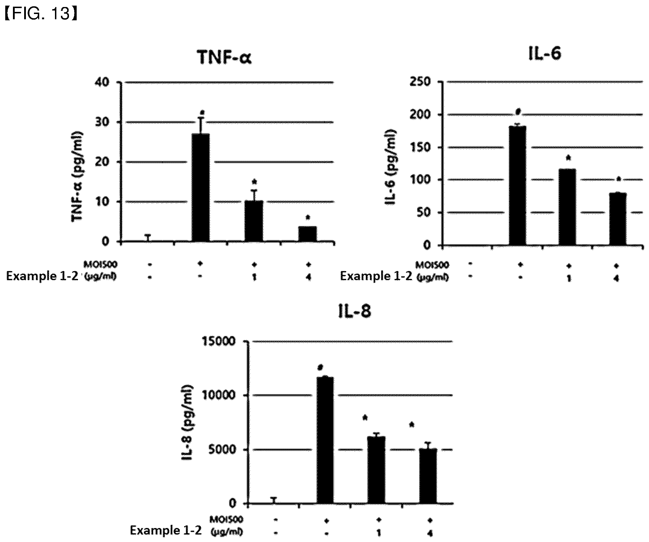

For the supernatant samples isolated from the cell culture plate, the amount of inflammatory cytokines secreted from nerve cells was measured by ELISA technique. The capture antibody was coated at an appropriate concentration in a 96 well plate, incubated at 25°C for 24 hours, and then washed three times with wash buffer. Blocking was performed using 300*µ*ℓ of Reagent Diluent buffer at 25°C for 1 hour, and washed three times with wash buffer. Then, the test sample and standard solution were treated by 100 µl each, and then incubated for 2 hours. All experimental groups were treated equally in two wells (duplicate) per group, and washed three times with wash buffer during treatment. The detection antibody was treated in 100 *µ*ℓ increments at an appropriate concentration, incubated for 2 hours, and then washed three times with wash buffer. After treating with 100*µ*ℓ of streptavidin-HRP, light was blocked for 20 minutes, and the cells were incubated at 25°C. After the incubation was completed, 50*µ*ℓ of stop solution was added and the absorbance (O.D₄₅₀ₙₘ) was measured. The result values were calculated from the average of the measured values, and the calculated amount of inflammatory cytokines is shown in FIG. 13.

As can be seen in FIGS. 12 and 13, it was confirmed that gene expression and production of major inflammatory cytokines (IL-6, IL-8, TNF-α) in nerve cells were suppressed by treatment with the example compound, thereby alleviating neuroinflammation.

### Example 8. Cytotoxicity evaluation

In order to confirm the safety of cells from the example compound, cytotoxicity against nerve cells used in the experiment was confirmed.

To evaluate whether the example compounds exhibit toxicity against nerve cells, human neuroblastoma (SH-SY5Y, 3×10⁵ cells/well) was dispensed into a cell culture plate (48-well plate), and then treated with the compound of Example 1-2 at concentrations of 2, 4, 8, 16, and 32 µM, and incubated at 37°C and 5% CO₂ incubator for 24 hours. After 24 hours, 50 µl of CCK-8 (cell counting kit-8, Dojindo Molecular Technologies, Inc) solution was added equally to each well, and then re-cultured in a 5% CO₂ incubator at 37°C for 1 to 3 hours. The cell activity was evaluated while measuring absorbance (O.D₄₅₀ₙₘ) at intervals of 1 hour of incubation. The cell viability of each experimental group was calculated based on the cell viability of the control group which was not treated with the example compound, considered as 100%, and the results are shown in FIG. 8.

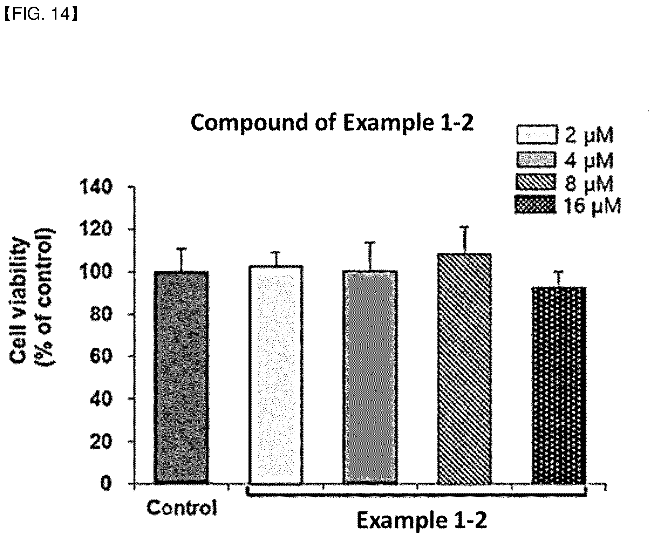

As can be seen in FIG. 14, the example compounds did not exhibit cytotoxicity in nerve cells at a concentration of 16 µM or less.

### Example 9. Results of blood-brain barrier permeation test

The compound of Example 1-2 was diluted to a concentration of 0.4 mg/ml in an excipient containing 10% DMSO + 65% PEG400 + 25% Sarin, and then injected intravenously (i.v.) at 5 ml per kg into male SD rats (Zhejiang Vital River Laboratory Animal Technology Co., Ltd). Blood samples were then collected in 60 µl increments at each time point (0.25 h, 0.5 h, 1 h, 5 h, 10 h). The collected blood samples were centrifuged at 8000 rpm at 4°C for 6 minutes, the supernatant was removed, plasma samples were collected at each time, and stored at -50°C until analysis, and then used for analysis.

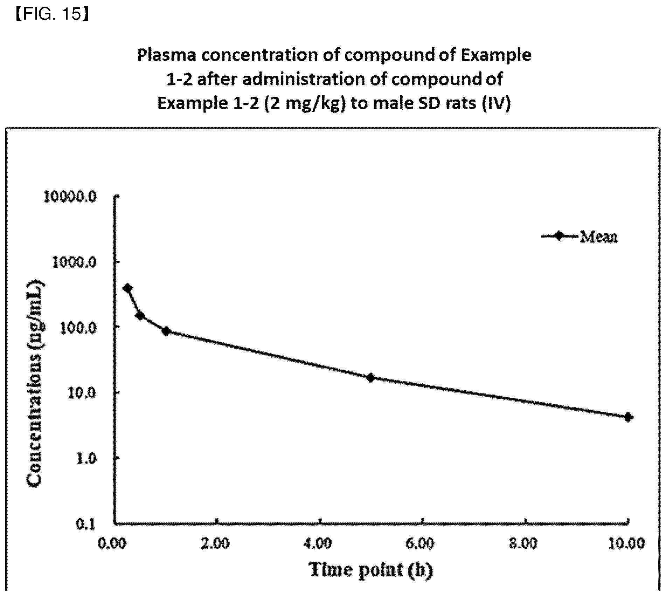

To analyze the concentration of substances in plasma, 20 µl of each hourly sample, standard sample, and QC (Quality Control) sample were mixed with 60 µl of acetonitrile (ACN) (Cas No. 75-05-8) containing an internal standard substance (tolbutamide (Cas No. 64-77-7) 200ng/ml, propranolol (Cas No. 318-98-9) 50ng/ml and dic(N,N'-diisopropylcarbodiimide) (Cas No. 693-13-0) 500 ng/ml). The mixture was vortexed for 1 minute, then centrifuged (13000 rpm, 4°C, 10 minutes), and the supernatant (50 µl) was isolated, transferred to a 96-well plate containing purified water (150 µl), and shaken and mixed for 10 minutes. The final mixture (3 µl) was injected into the LC-MS/MS system to analyze the concentration of compounds in the plasma sample in each time zone, and the results are shown in FIG. 15.

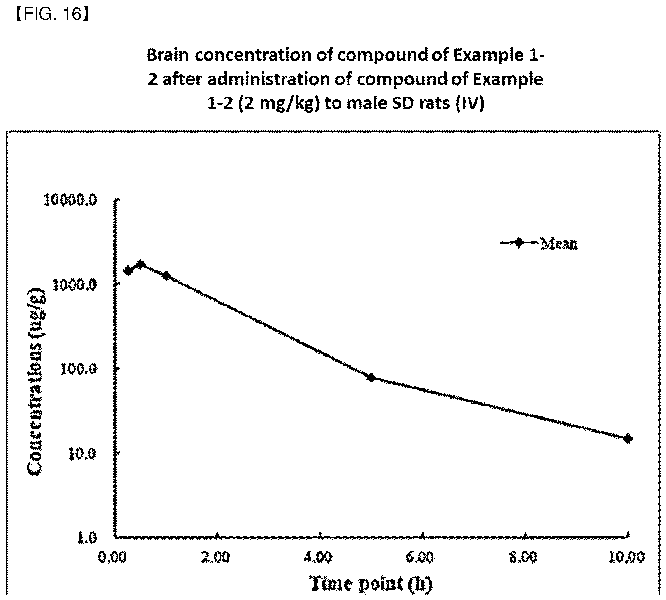

In the case of a brain sample, in order to obtain only the sample in the brain tissue, heart perfusion was performed and then the brain tissue was isolated. Three times the weight (w/v) of saline solution was added to the isolated brain tissue, which was then ground together to prepare a homogenized sample solution. After 20µl of each brain sample, standard sample, and QC sample were mixed with 60µl of acetonitrile (ACN) (Cas No. 75-05-8) containing the internal standard material (tolbutamide (Cas No. 64-77-7) 200ng/ml, propranolol (Cas No. 318-98-9) 50ng/ml, and dic(N,N'-diisopropylcarbodiimide) (Cas No. 693)-13-0) 500ng/ml), and the mixture was vortexed for 1 minute, centrifuged (13000 rpm, 4 °C, 10 min), and the supernatant (50 µl) was isolated and transferred to a 96-well plate containing purified water (150 µl), and then shaken and mixed for 10 minutes. The final mixture (3 µl) was injected into the LC-MS/MS system to analyze the concentration of the compound in the brain sample at each time zone. The concentration of the compound in the brain tissue was analyzed by converting the calculated sample solution concentration (ng/ml) into the concentration in the brain tissue (ng/g) reflecting the dilution factor, and the results are shown in FIG. 16.

As can be seen in FIGS. 15 and 16, it was confirmed that when the example compound was administered intravenously (i.v.) at a dose of 2 mg/kg to male SD rats, the example compound passed through the blood and then the blood-brain barrier, and reached the brain, and the example compounds remained in the brain up to 10 hours after administration. Through the above results, it was confirmed that the example compound acts on the lesion in the brain which is the target site, and can treat neurodegenerative diseases.

### Example 10. Confirmation of anti-inflammatory effect in macrophages

In order to confirm the inflammation-reducing effect by the example compounds, mouse bone marrow-derived macrophages were differentiated, and the changes in the production of inflammatory cytokine (IL-1β) in macrophages upon treatment with the compounds were measured.

Bone marrow was isolated from the femur and tibia of 8-12 week old C57BU6J mice (available from Daehan Biolink) using a 26 g syringe, and the isolated bone marrow was differentiated into macrophages by culturing a medium containing IMDM media + 30% L929 cell supernatnant + 10% fetal bovine serum + 1% pencillin/streptomycin + 1% non-essential amino acid + 1% sodium pyruvate in a 150π cell culture plate for 6 days, and used for the experiment.

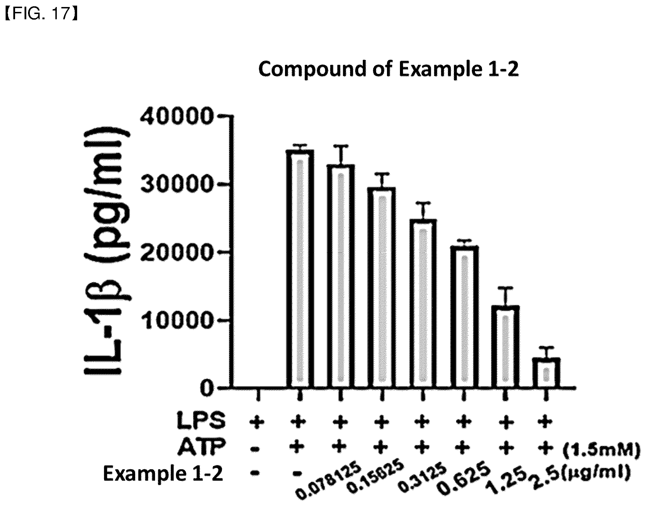

The differentiated macrophages were seeded in 200 µl increments in a 48-well plate at a concentration of 1 ×10⁶ cell/ml, and stabilized at 37°C and 5% CO₂ for 12 hours. After the supernatant of the cultured cells was removed, 180 µl of LPS was treated onto the serum free medium at a concentration of 100 ng/ml, which was primed for 5 hours. The compound of Example 1-2 was diluted to concentrations of 0.78125, 1.5625, 3.125, 6.25, 12.5, and 25 mg/ml and added in 200 µl increments (working concentration: 0.078125, 0.15625, 0.3125, 0.625, 1.25, 2.5 mg/ml). After 1 hour, ATP (added by 20 µl at 10 mM concentration, incubated for 30 minutes) was treated according to the conditions. After the cultured cell supernatant sample was collected, the amount of IL-1β, which is a representative inflammatory cytokine and a cytokine released by NLRP3 inflammasomes activity, was measured using the ELISA technique, and is shown in FIG. 17.

As can be seen in FIG. 17, it was confirmed that the IL-1β release increased by a stimulation source in macrophages was inhibited in a concentration-dependent manner by the compounds of Examples 1 and 2. Therefore, the inflammatory response induced in macrophages is inhibited by the example compound.

### Example 11. Cytotoxicity evaluation

In order to confirm the safety of cells from the example compound, it was evaluated whether the compound exhibited cytotoxicity in mouse bone marrow-derived macrophages and human monocyte cell line (THP-1) (available from Korea Cell Line Bank (KCLB)).

In the case of macrophages, the differentiated macrophages were seeded in 200 µl increments in a 48-well plate at a concentration of 1×10⁶ cell/ml, and stabilized at 37°C and 5% CO₂ for 12 hours. After the supernatant of the cultured cells was removed, the compound of Example 1-2 were diluted to concentrations of 0.5, 1, 2, 4, 8, and 16 µM in a serum free medium, treated in 200 µl increments, and cultured at 37°C and 5% CO₂ for 24 hours.

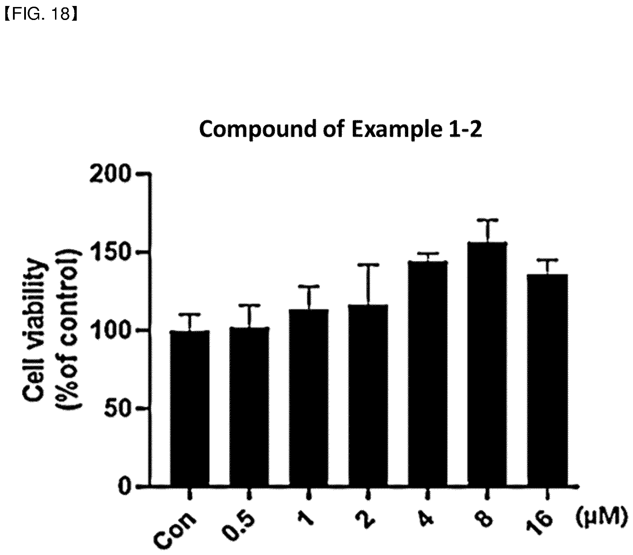

The supernatant of the cultured cells was then removed, 200 µl of MTT solution having a concentration of 400 µg/ml was added, and then reacted at 7°C and 5% CO₂ for 4 hours. Then, the reaction solution was removed, 200 µl of DMSO was added, and the absorbance was measured at 570 nm. At this time, the viability of cells treated with the sample was calculated based on the viability of untreated cells considered as 100%, and is shown in FIG. 18.

The human monocyte cell line(THP-1) was cultured in a 10% fetal bovine serum (Hyclone, Waltham, MA, USA), and a RPMI 1640 medium supplemented with 2.05 mM L-glutamate and antibiotics (100 units/ml penicillin, 100 µg/ml streptomycin), and then seeded in a 96 well microtiter plate at a concentration of 1×10⁵ cells/well. The compound of Example 1-2 was diluted to a final concentration of 0.002 µM, 0.02 µM, 0.2 µM, and 2 µM, cells in each well were treated and cultured for 24 hours.

Then, the cultured cells were treated with 10 µl of Cell Counting Kit-8 (CCK-8, DOJINDO, Kumamoto, Japan) solution in each well, and then the absorbance was measured at 450 nm for each time zone (1 h, 2 h, 3 h), and the results were derived based on the absorbance data from the last time zone, 3 h.

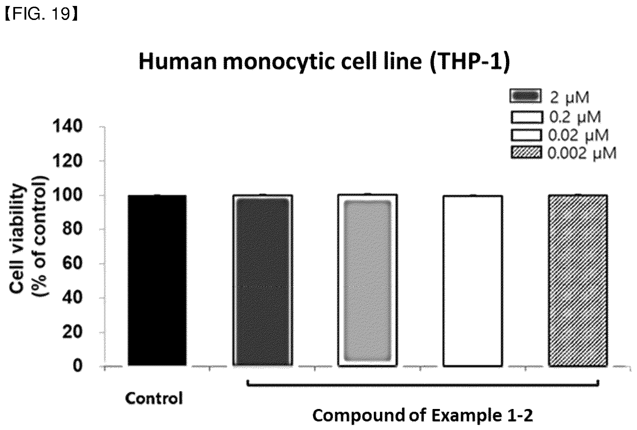

At this time, the cell viability of untreated cells was set as 100% as the reference, and the cell viability of the cells treated with the sample was calculated and shown in FIG. 19.

As can be seen in FIGS. 18 and 19, the compound of Example 1-2 did not exhibit cytotoxicity at a concentration of 16 µM or less in mouse bone marrow-derived macrophages, and also did not exhibit cytotoxicity at any concentration in human monocyte cell line (THP-1).

### Example 12. Evaluation of drug safety through rat micronucleus test

In order to confirm the safety of the example compounds in laboratory animals, i.e., rats (purchased by ORIENTBIO INC., Republic of Korea), Sprague-Dawley rat bone marrow cells were used to general symptoms, body weight changes and presence/absence of micronucleus induction (genotoxicity) after administration of the substance.

Hair was removed from the administration site (subcutaneous) of the SD rats, the administration concentration of the example compounds were set to a low dose(500mg/kg/day), a medium dose(1000mg/kg/day), and a high dose(2000mg/kg/day), and administered subcutaneously to the left and right cervixes of the test system (10 ml/kg/day each), and the compound was administered subcutaneously in divided doses to the left and right cervix sites of the test system (10 ml/kg/day each). During the administration period, twice per day (immediately before administration and immediately after administration), once per day during the rest of the period, general symptoms such as appearance, behavior, and excretion of each individual were observed, and the results are shown in Table 3 below.

In addition, body weight was measured on the day of administration start and bone marrow collection to confirm presence/absence of changes due to administration of the substance, and the results are shown in Table 4 below.

24 hours after the second administration of the test substance, the femur was removed from each group to collect bone marrow cells. 0.5 mL of 10% neutral formalin was added to the bone marrow cell suspension, fixed for 5 minutes, centrifuged for 5 minutes (4°C, 1,000 rpm, Micro17TR, Hanil Science Industrial, Republic of Korea) to remove the supernatant. Then, 0.3 mL of 10% neutral formalin was added to the precipitated bone marrow cells, suspended, filtered through a cell strain, and transferred to a storage tube. The fixed bone marrow cell suspension was dropped onto a cover glass, and then covered with a slide glass coated with 20 µL of 0.05% acridine orange to prepare an observation slide. The observation slide was observed under a microscope (600x magnification, BX51, Olympus, Japan). 4,000 polychromatic erythrocytes(PCE) per individual were observed, and the occurrence ratio of micronucleated polychromatic erythrocytes(MNPCE) to polychromatic erythrocytes (MNPCE/PCE) was calculated for each individual, and the results are shown in Table 3 below.

In addition, it was confirmed from the observation results that the ratio of polychromatic red blood cells to the total number of red blood cells in the test substance group (treated with the compound of Example 1-2) exhibited to be 20% or more of the negative control. As an indicator of bone marrow cell proliferation inhibition, 500 total red blood cells per individual were observed, and the ratio of polychromatic red blood cells to total red blood cells (PCE/(PCE+NCE)) was calculated. The results are shown in Table 5 below. As a positive control, cyclophosphamide (Cas No: 50-18-0) was administered at a dose of 20 mg/kg/day.

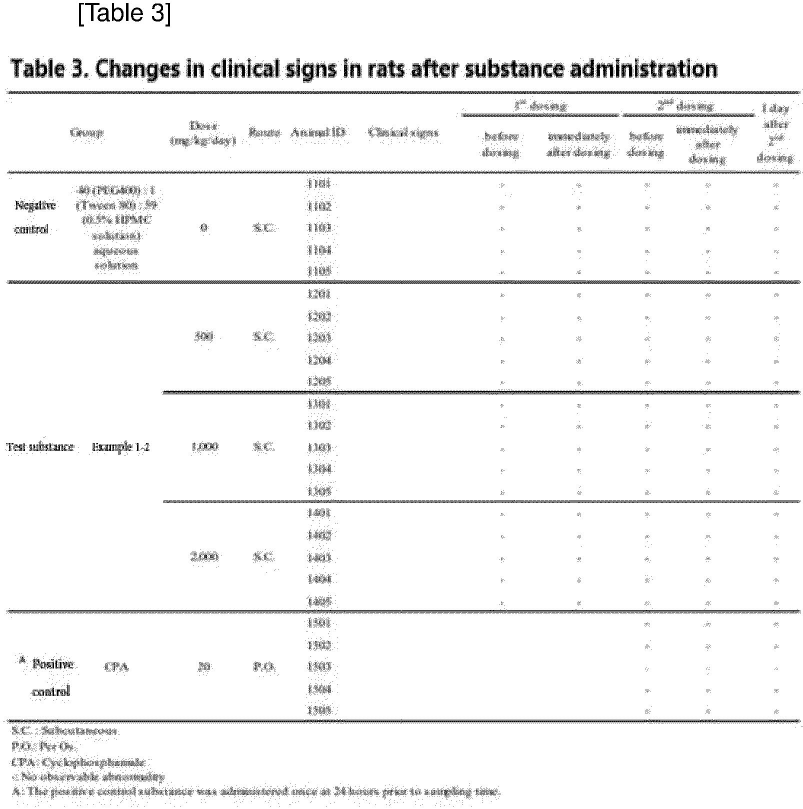

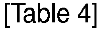

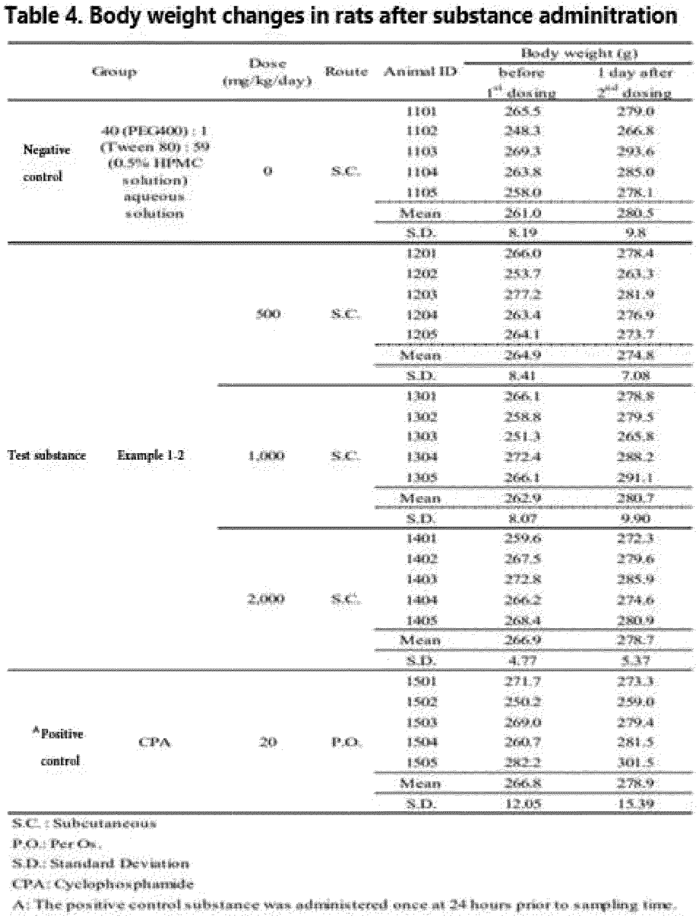

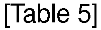

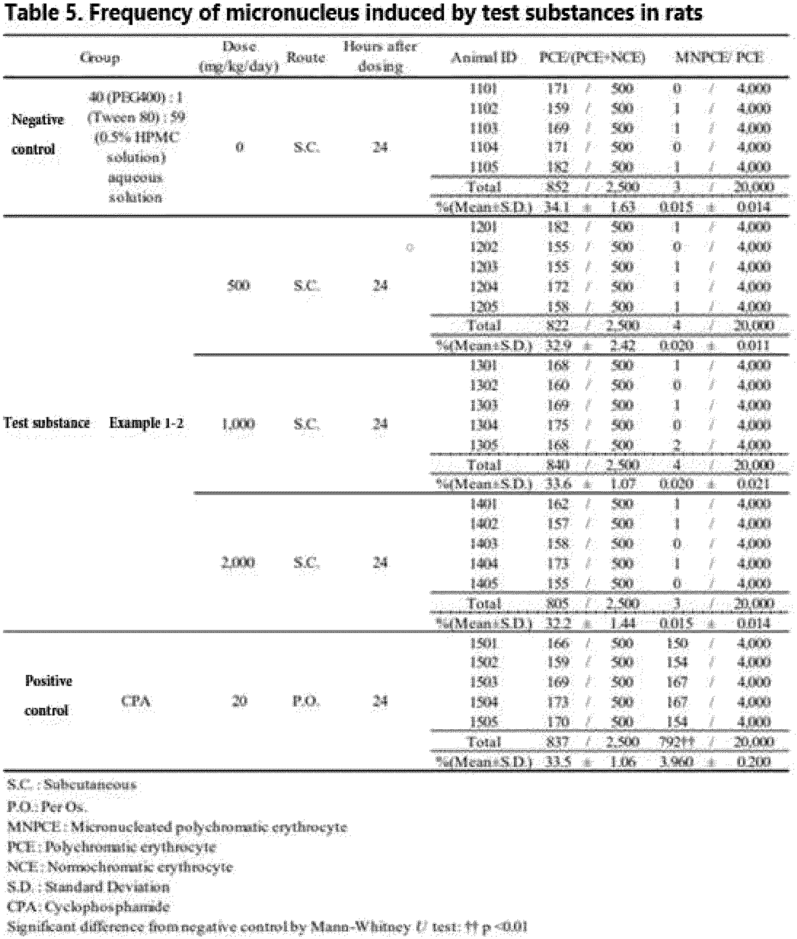

As can be seen from the experimental results in Table 3, at all doses of the compound of Example 1-2 during the observation period, no abnormal clinical signs in rats were observed in the test substance group (the group administered with the compound of Example 1-2).

Further, as can be seen in Table 4, at all doses of the compounds of Example 1-2, the test substance group did not show a statistically significant change in body weight compared to the negative control.

Further, as can be seen in Table 5, at all doses of the compound of Example 1-2, the test substance group did not show a statistically significant difference in the appearance frequency of micronucleated polychromatic erythrocytes (MNPCE/PCE) in polychromatic erythrocytes compared to the negative control, and also did not show a statistically significant difference in the ratio of polychromatic red blood cells in total red blood cells [PCE/(PCE+NCE)] compared to the negative control. On the other hand, it was not confirmed that the ratio of polychromatic red blood cells was statistically significantly different from that in the negative control, but it was confirmed that the appearance frequency of micronucleated polychromatic erythrocytes in polychromatic erythrocytes was statistically significantly increased compared to the negative control (p < 0.01).

From the above results, it was confirmed that the example compounds were safe as they did not induce micronuclei in rat bone marrow cells.

From the above description, those skilled in the art to which the present application pertains will be able to understand that the present application may be executed in other specific forms without changing the technical spirit or essential characteristics thereof. In this regard, it should be understood that Examples described above are illustrative and not restrictive in all respects. The scope of the present application should be construed as that all changed or modified forms derived from the meaning and scope of claims to be described below and equivalent concepts rather than the detailed description are included in the scope of the present application.

## Claims

1. A pharmaceutical composition for preventing or treating inflammasome-mediated disease, comprising at least one selected from the group consisting of a compound of the following Chemical Formula 1, an isomer thereof, and a pharmaceutically acceptable salt thereof as an active ingredient: 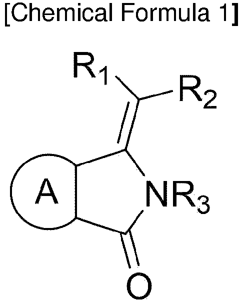 wherein in Chemical Formula 1,
the 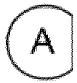 is a substituted or unsubstituted C₃₋₇ cycloalkyl, a substituted or unsubstituted C₃₋₇ cycloalkenyl containing at least one double bond, a substituted or unsubstituted C₃₋₇ heterocycloalkyl containing at least one heteroatom selected from the group consisting of N, O, and S, a substituted or unsubstituted C₃₋₇ heterocycloalkenyl containing at least one double bond and containing at least one heteroatom selected from the group consisting of N, O, and S, a substituted or unsubstituted C₆₋₁₀ aryl, or a substituted or unsubstituted C₅₋₁₀ heteroaryl containing at least one heteroatom selected from the group consisting of N, O, and S,
the substituted cycloalkyl, the substituted cycloalkenyl, the substituted heterocycloalkyl, the substituted heterocycloalkenyl, the substituted aryl, or the substituted heteroaryl may be substituted with at least one substituent selected from the group consisting of hydroxy, halogen, cyano, nitro, amino, a substituted or unsubstituted C₁₋₁₀ linear or branched alkyl, and a substituted or unsubstituted C₁₋₁₀ linear or branched alkoxy,
the substituted alkyl or the substituted alkoxy may be substituted with at least one substituent selected from the group consisting of hydroxy, halogen, cyano, nitro, amino, a C₁₋₅ linear or branched alkyl, and a C₁₋₅ linear or branched alkoxy,
the R₃ is a substituted or unsubstituted C₁₋₁₀ linear or branched alkyl, a substituted or unsubstituted C₁₋₁₀ linear or branched chain alkoxy, a substituted or unsubstituted C₃₋₇ cycloalkyl, a substituted or unsubstituted C₃₋₇ heterocycloalkyl containing at least one heteroatom selected from the group consisting of N, O, and S, a substituted or unsubstituted C₆₋₁₀ aryl, or a substituted or unsubstituted C₅₋₁₀ heteroaryl containing at least one heteroatom selected from the group consisting of N, O, and S,
the substituted alkyl may be substituted with at least one substituent selected from the group consisting of hydroxy, halogen, cyano, nitro, amino, a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing one or more heteroatoms selected from the group consisting of N, O, and S,
the substituted alkoxy, the substituted cycloalkyl, the substituted heterocycloalkyl, the substituted aryl, or the substituted heteroaryl may be substituted with at least one substituent selected from the group consisting of hydroxy, halogen, cyano, nitro, amino, a C₁₋₅ linear or branched alkyl, and a C₁₋₅ linear or branched alkoxy,
the R₁ and R₂ are each independently hydrogen, halogen, a substituted or unsubstituted C₁₋₁₀ linear or branched alkyl, a substituted or unsubstituted C₁₋₁₀ linear or branched alkoxy, a substituted or unsubstituted C₆₋₁₀ aryl, or a substituted or unsubstituted C₅₋₁₀ heteroaryl containing at least one heteroatom selected from the group consisting of N, O, and S,
the substituted alkyl may be substituted with at least one substituent selected from the group consisting of hydroxy, halogen, cyano, nitro, amino, a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing at least one heteroatom selected from the group consisting of N, O, and S, and
the substituted alkoxy, the substituted aryl, or the substituted heteroaryl may be substituted with at least one substituent selected from the group consisting of hydroxy, halogen, cyano, nitro, amino, a C₁₋₅ linear or branched alkyl, and a C₁₋₅ linear or branched alkoxy.

2. The pharmaceutical composition according to claim 1, wherein the 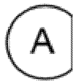 is a substituted or unsubstituted C₆₋₁₀ aryl,
the substituted aryl may be substituted with at least one substituent selected from the group consisting of hydroxy, halogen, cyano, nitro, amino, a substituted or unsubstituted C₁₋₁₀ linear or branched alkyl, and a substituted or unsubstituted C₁₋₁₀ linear or branched alkoxy, and
the substituted alkyl or the substituted alkoxy may be substituted with at least one substituent selected from the group consisting of hydroxy, halogen, cyano, nitro, amino, a C₁₋₅ linear or branched alkyl, and a C₁₋₅ linear or branched alkoxy.

3. The pharmaceutical composition according to claim 1, wherein the R₃ is a substituted or unsubstituted C₁₋₁₀ linear or branched alkyl.

4. The pharmaceutical composition according to claim 1, wherein the R₁ and R₂ are each independently a substituted or unsubstituted C₆₋₁₀ aryl, and
the substituted aryl may be substituted with at least one substituent selected from the group consisting of hydroxy, halogen, cyano, nitro, amino, a C₁₋₅ linear or branched alkyl, and a C₁₋₅ linear or branched alkoxy.

5. The pharmaceutical composition according to claim 1, wherein the compound of Chemical Formula 1 is selected from the group consisting of the following compounds:
3-(diphenylmethylene)-2-methylisoindolin-1-one,
3-(di-p-tolylmethylene)-2-methylisoindolin-1-one, and
3-(bis(4-methoxyphenyl)methylene)-2-methylisoindolin-1-one.

6. The pharmaceutical composition according to claim 1, wherein the inflammasome-mediated disease is at least one selected from the group consisting of a periodontal disease, a neurodegenerative disease, and an inflammatory disease.

7. The pharmaceutical composition according to claim 6, wherein the periodontal disease is at least one selected from the group consisting of periodontitis and gingivitis.

8. The pharmaceutical composition according to claim 6, wherein the neurodegenerative disease is at least one selected from the group consisting of dementia, Parkinson's disease, hand tremor, chorea, proximal lateral sclerosis, multiple sclerosis, Huntington's disease, motor neuron disease, spinal muscular atrophy, Creutzfeldt-Jakob disease, Pick's disease, Prion disease and spinocerebellar ataxia.

9. The pharmaceutical composition according to claim 6, wherein the inflammatory disease is at least one selected from the group consisting of
at least one metabolic disease selected from the group consisting of obesity, hyperlipidemia, hypercholesterolemia, arteriosclerosis, type 2 diabetes, non-alcoholic fatty liver disease(NAFLD) and non-alcoholic steatohepatitis(NASH);
at least one autoinflammatory disease selected from the group consisting of Muckle-Wells syndrome(MWS), latent autoimmune diabetes in adults(LADA), familial cold autoinflammatory syndrome(FCAS), cryopyrin-associated periodic syndrome(CAPS); neonatal-onset multisystem inflammatory syndrome(NOMID), chronic infantile neurocutaneous articular(CINCA) syndrome, Familial Mediterranean fever(FMF), juvenile arthritis, juvenile rheumatoid arthritis and gout;
at least one autoimmune diseases selected from the group consisting of rheumatoid arthritis(RA), systemic lupus erythematosus(SLE), atopic dermatitis(AD) and psoriasis;
at least one disease selected from the group consisting of septic shock, inflammatory bowel disease, osteoarthritis, hyperimmunoglobulin D syndrome and cryopyrin-associated periodic syndromes.

10. A food composition for preventing or alleviating inflammasome-mediated disease, comprising at least one selected from the group consisting of the compound of Chemical Formula 1 according to any one of claims 1 to 5, an isomer thereof, and a pharmaceutically acceptable salt thereof as an active ingredient.

11. A feed composition for preventing or alleviating inflammasome-mediated disease, comprising at least one selected from the group consisting of the compound of Chemical Formula 1 according to any one of claims 1 to 5, an isomer thereof, and a pharmaceutically acceptable salt thereof as an active ingredient.

12. A pharmaceutical composition for preventing or treating disease caused by Gram-negative bacterial infection or Gram-negative bacterium, comprising at least one selected from the group consisting of a compound of the following Chemical Formula 1, an isomer thereof, and a pharmaceutically acceptable salt thereof as an active ingredient: 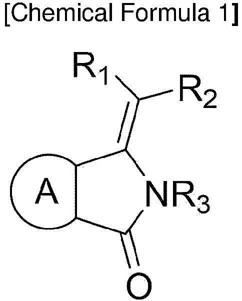 wherein in Chemical Formula 1,
the 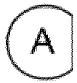 is a substituted or unsubstituted C₃₋₇ cycloalkyl, a substituted or unsubstituted C₃₋₇ cycloalkenyl containing at least one double bond, a substituted or unsubstituted C₃₋₇ heterocycloalkyl containing at least one heteroatom selected from the group consisting of N, O, and S, a substituted or unsubstituted C₃₋₇ heterocycloalkenyl containing at least one double bond and containing at least one heteroatom selected from the group consisting of N, O, and S, a substituted or unsubstituted C₆₋₁₀ aryl, or a substituted or unsubstituted C₅₋₁₀ heteroaryl containing at least one heteroatom selected from the group consisting of N, O, and S,
the substituted cycloalkyl, the substituted cycloalkenyl, the substituted heterocycloalkyl, the substituted heterocycloalkenyl, the substituted aryl, or the substituted heteroaryl may be substituted with at least one substituent selected from the group consisting of hydroxy, halogen, cyano, nitro, amino, a substituted or unsubstituted C₁₋₁₀ linear or branched alkyl, and a substituted or unsubstituted C₁₋₁₀ linear or branched alkoxy,
the substituted alkyl or the substituted alkoxy may be substituted with at least one substituent selected from the group consisting of hydroxy, halogen, cyano, nitro, amino, a C₁₋₅ linear or branched alkyl, and a C₁₋₅ linear or branched alkoxy,
the R₃ is a substituted or unsubstituted C₁₋₁₀ linear or branched alkyl, a substituted or unsubstituted C₁₋₁₀ linear or branched chain alkoxy, a substituted or unsubstituted C₃₋₇ cycloalkyl, a substituted or unsubstituted C₃₋₇ heterocycloalkyl containing at least one heteroatom selected from the group consisting of N, O, and S, a substituted or unsubstituted C₆₋₁₀ aryl, or a substituted or unsubstituted C₅₋₁₀ heteroaryl containing at least one heteroatom selected from the group consisting of N, O, and S,
the substituted alkyl may be substituted with at least one substituent selected from the group consisting of hydroxy, halogen, cyano, nitro, amino, a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing one or more heteroatoms selected from the group consisting of N, O, and S,
the substituted alkoxy, the substituted cycloalkyl, the substituted heterocycloalkyl, the substituted aryl, or the substituted heteroaryl may be substituted with at least one substituent selected from the group consisting of hydroxy, halogen, cyano, nitro, amino, a C₁₋₅ linear or branched alkyl, and a C₁₋₅ linear or branched alkoxy,
the R₁ and R₂ are each independently hydrogen, halogen, a substituted or unsubstituted C₁₋₁₀ linear or branched alkyl, a substituted or unsubstituted C₁₋₁₀ linear or branched alkoxy, a substituted or unsubstituted C₆₋₁₀ aryl, or a substituted or unsubstituted C₅₋₁₀ heteroaryl containing at least one heteroatom selected from the group consisting of N, O, and S,
the substituted alkyl may be substituted with at least one substituent selected from the group consisting of hydroxy, halogen, cyano, nitro, amino, a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing at least one heteroatom selected from the group consisting of N, O, and S, and
the substituted alkoxy, the substituted aryl, or the substituted heteroaryl may be substituted with at least one substituent selected from the group consisting of hydroxy, halogen, cyano, nitro, amino, a C₁₋₅ linear or branched alkyl, and a C₁₋₅ linear or branched alkoxy.

13. The pharmaceutical composition according to claim 12, wherein the 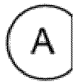 is a substituted or unsubstituted C₆₋₁₀ aryl,
the substituted aryl may be substituted with at least one substituent selected from the group consisting of hydroxy, halogen, cyano, nitro, amino, a substituted or unsubstituted C₁₋₁₀ linear or branched alkyl, and a substituted or unsubstituted C₁₋₁₀ linear or branched alkoxy, and
the substituted alkyl or the substituted alkoxy may be substituted with at least one substituent selected from the group consisting of hydroxy, halogen, cyano, nitro, amino, a C₁₋₅ linear or branched alkyl, and a C₁₋₅ linear or branched alkoxy.

14. The pharmaceutical composition according to claim 12, wherein the R₃ is a substituted or unsubstituted C₁₋₁₀ linear or branched alkyl.

15. The pharmaceutical composition according to claim 12, wherein the R₁ and R₂ are each independently a substituted or unsubstituted C₆₋₁₀ aryl, and
the substituted aryl may be substituted with at least one substituent selected from the group consisting of hydroxy, halogen, cyano, nitro, amino, a C₁₋₅ linear or branched alkyl, and a C₁₋₅ linear or branched alkoxy.

16. The pharmaceutical composition according to claim 12, wherein the compound of Chemical Formula 1 is selected from the group consisting of the following compounds:
3-(diphenylmethylene)-2-methylisoindolin-1-one,
3-(di-p-tolylmethylene)-2-methylisoindolin-1-one, and
3-(bis(4-methoxyphenyl)methylene)-2-methylisoindolin-1-one.

17. The pharmaceutical composition according to claim 12, wherein the disease caused by Gram-negative bacterial infection or Gram-negative bacterium is at least one selected from the group consisting of enteritis, Crohn's disease, ulcerative colitis, bacillary dysentery, urinary tract infection, skin infection, bacteremia, sepsis, skin infections, bedsores, pneumonia, endocarditis, meningitis, otitis externa, otitis media, periodontitis, gingivitis, keratitis, osteomyelitis, peritonitis, and cystic fibrosis.

18. A food composition for preventing or alleviating disease caused by Gram-negative bacterial infection or Gram-negative bacterium, comprising at least one selected from the group consisting of the compound of Chemical Formula 1 according to any one of claims 12 to 17, an isomer thereof, and a pharmaceutically acceptable salt thereof as an active ingredient.

19. A feed composition for preventing or alleviating disease caused by Gram-negative bacterial infection or Gram-negative bacterium, comprising at least one selected from the group consisting of the compound of Chemical Formula 1 according to any one of claims 12 to 17, an isomer thereof, and a pharmaceutically acceptable salt thereof as an active ingredient.

20. An antibiotic comprising at least one selected from the group consisting of a compound of the following Chemical Formula 1, an isomer thereof, and a pharmaceutically acceptable salt thereof as an active ingredient: 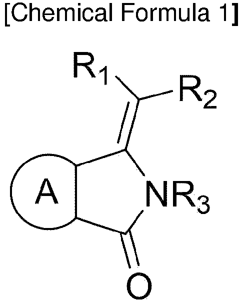 wherein in Chemical Formula 1,
the 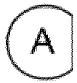 is a substituted or unsubstituted C₃₋₇ cycloalkyl, a substituted or unsubstituted C₃₋₇ cycloalkenyl containing at least one double bond, a substituted or unsubstituted C₃₋₇ heterocycloalkyl containing at least one heteroatom selected from the group consisting of N, O, and S, a substituted or unsubstituted C₃₋₇ heterocycloalkenyl containing at least one double bond and containing at least one heteroatom selected from the group consisting of N, O, and S, a substituted or unsubstituted C₆₋₁₀ aryl, or a substituted or unsubstituted C₅₋₁₀ heteroaryl containing at least one heteroatom selected from the group consisting of N, O, and S,
the substituted cycloalkyl, the substituted cycloalkenyl, the substituted heterocycloalkyl, the substituted heterocycloalkenyl, the substituted aryl, or the substituted heteroaryl may be substituted with at least one substituent selected from the group consisting of hydroxy, halogen, cyano, nitro, amino, a substituted or unsubstituted C₁₋₁₀ linear or branched alkyl, and a substituted or unsubstituted C₁₋₁₀ linear or branched alkoxy,
the substituted alkyl or the substituted alkoxy may be substituted with at least one substituent selected from the group consisting of hydroxy, halogen, cyano, nitro, amino, a C₁₋₅ linear or branched alkyl, and a C₁₋₅ linear or branched alkoxy,
the R₃ is a substituted or unsubstituted C₁₋₁₀ linear or branched alkyl, a substituted or unsubstituted C₁₋₁₀ linear or branched chain alkoxy, a substituted or unsubstituted C₃₋₇ cycloalkyl, a substituted or unsubstituted C₃₋₇ heterocycloalkyl containing at least one heteroatom selected from the group consisting of N, O, and S, a substituted or unsubstituted C₆₋₁₀ aryl, or a substituted or unsubstituted C₅₋₁₀ heteroaryl containing at least one heteroatom selected from the group consisting of N, O, and S,
the substituted alkyl may be substituted with at least one substituent selected from the group consisting of hydroxy, halogen, cyano, nitro, amino, a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing one or more heteroatoms selected from the group consisting of N, O, and S,
the substituted alkoxy, the substituted cycloalkyl, the substituted heterocycloalkyl, the substituted aryl, or the substituted heteroaryl may be substituted with at least one substituent selected from the group consisting of hydroxy, halogen, cyano, nitro, amino, a C₁₋₅ linear or branched alkyl, and a C₁₋₅ linear or branched alkoxy,
the R₁ and R₂ are each independently hydrogen, halogen, a substituted or unsubstituted C₁₋₁₀ linear or branched alkyl, a substituted or unsubstituted C₁₋₁₀ linear or branched alkoxy, a substituted or unsubstituted C₆₋₁₀ aryl, or a substituted or unsubstituted C₅₋₁₀ heteroaryl containing at least one heteroatom selected from the group consisting of N, O, and S,
the substituted alkyl may be substituted with at least one substituent selected from the group consisting of hydroxy, halogen, cyano, nitro, amino, a C₁₋₅ linear or branched alkyl, and a C₁₋₅ linear or branched alkoxy; or may be substituted with a substituted or unsubstituted C₆₋₁₀ aryl, or a substituted or unsubstituted C₅₋₁₀ heteroaryl containing at least one heteroatom selected from the group consisting of N, O, and S, and
the substituted alkoxy, the substituted aryl, or the substituted heteroaryl may be substituted with at least one substituent selected from the group consisting of hydroxy, halogen, cyano, nitro, amino, a C₁₋₅ linear or branched alkyl, and a C₁₋₅ linear or branched alkoxy.

21. The antibiotic according to claim 20, wherein the 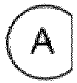 is a substituted or unsubstituted C₆₋₁₀ aryl,
the substituted aryl may be substituted with at least one substituent selected from the group consisting of hydroxy, halogen, cyano, nitro, amino, a substituted or unsubstituted C₁₋₁₀ linear or branched alkyl, and a substituted or unsubstituted C₁₋₁₀ linear or branched alkoxy, and
the substituted alkyl or the substituted alkoxy may be substituted with at least one substituent selected from the group consisting of hydroxy, halogen, cyano, nitro, amino, a C₁₋₅ linear or branched alkyl, and a C₁₋₅ linear or branched alkoxy.

22. The antibiotic according to claim 20, wherein the R₃ is a substituted or unsubstituted C₁₋₁₀ linear or branched alkyl.

23. The antibiotic according to claim 20, wherein the R₁ and R₂ are each independently a substituted or unsubstituted C₆₋₁₀ aryl, and
the substituted aryl may be substituted with at least one substituent selected from the group consisting of hydroxy, halogen, cyano, nitro, amino, a C₁₋₅ linear or branched alkyl, and a C₁₋₅ linear or branched alkoxy.

24. The antibiotic according to claim 20, wherein the compound of Chemical Formula 1 is selected from the group consisting of the following compounds:
3-(diphenylmethylene)-2-methylisoindolin-1-one,
3-(di-p-tolylmethylene)-2-methylisoindolin-1-one, and
3-(bis(4-methoxyphenyl)methylene)-2-methylisoindolin-1-one.

25. The antibiotic according to claim 20, which has antibiotic activity against Gram-negative bacterium.

26. The antibiotic according to claim 20, wherein the Gram-negative bacterium is at least one selected from the group consisting of Escherichia sp. bacterium, Pseudomonas sp. bacterium, Acinetobacter sp. bacterium, Enterobacter sp. bacterium, Klebsiella sp. bacterium, Porphyromonas sp. bacterium, Fusobacteria sp. bacterium, and Tannerella sp. bacterium.

27. A combination antibiotic comprising:
the antibiotic according to claim 20; and
at least one second antibiotic selected from the group consisting of betalactam antibiotics, bacterial cell membrane permeability inhibitors, bacterial ribosome inhibitors, bacterial nucleic acid synthesis inhibitors, and bacterial folate synthesis inhibitors.

28. A feed additive comprising the antibiotic according to claim 20.

29. A disinfectant comprising the antibiotic according to claim 20.

30. A cleaning agent comprising the antibiotic according to claim 20.

31. A compound of the following Chemical Formula 1A, an isomer thereof, or a pharmaceutically acceptable salt thereof: 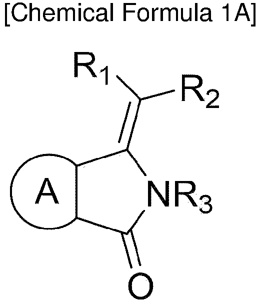 wherein in Chemical Formula 1A,
the 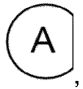 R₁, R₂ and R₃ are as defined in claim 1, and
the compound of Chemical formula 1A do not include the following compound:
3-(diphenylmethylene)-2-methylisoindolin-1-one.

32. The compound, the isomer thereof, or the pharmaceutically acceptable salt thereof according to claim 31, wherein the compound of Chemical Formula 1A is selected from the group consisting of the following compounds:
3-(di-p-tolylmethylene)-2-methylisoindolin-1-one, and
3-(bis(4-methoxyphenyl)methylene)-2-methylisoindolin-1-one.

33. A method for preparing the compound according to claim 31 or 32, the method comprising introducing phenylboronic acid (Cas No. 98-80-6), 4-methylphenylboronic acid (Cas No. 5720-05-8), or (4-methoxyphenyl)boronic acid (Cas No. 5720-07-0) into a compound of the following Chemical Formula 4:
